# EUROPEAN PATENT SPECIFICATION

(11) **EP 2 688 582 B1**
(45) Date of publication and mention of the grant of the patent: **15.02.2017**
(21) Application number: 12716661.9
(22) Date of filing: 22.03.2012
(51) Int. Cl.: A61K 38/17, A61P 25/28

(54) **NEUROPROTECTIVE PEPTIDES**
NEUROPROTEKTIVE PEPTIDE
PEPTIDES NEUROPROTECTEURS

(30) Priority: 24.03.2011 US 201161466966 P
(43) Date of publication of application: 29.01.2014
(73) Proprietor: NEURIM PHARMACEUTICALS (1991) LIMITED, Tel Aviv 69710 (IL)
(72) Inventor: PINNER, Elhanan, 42920 Moshav Beit Yitzhak (IL); ZISAPEL, Nava, 69865 Tel-Aviv (IL)
(74) Representative: V.O.
(86) International application number: PCT/IL2012/050104
(87) International publication number: WO 2012/127475

(56) References cited:
- WO-A2-2009/007934
- PINNER E ET AL: "CD44V10 plays a role in Abeta-induced neuronal cell death", ALZHEIMER'S & DEMENTIA: THE JOURNAL OF THE ALZHEIMER'SASSOCIATION, ELSEVIER, NEW YORK, NY, US, vol. 6, no. 4, 1 July 2010 (2010-07-01), pages S251-S252, XP027439338, ISSN: 1552-5260, DOI: 10.1016/J.JALZ.2010.05.820 [retrieved on 2010-07-01]
- PATRICIA KAAIJK ET AL: "Differential expression of CD44 splice variants in the normal human central nervous system", JOURNAL OF NEUROIMMUNOLOGY, vol. 73, no. 1-2, 1 March 1997 (1997-03-01), pages 70-76, XP55035954, ISSN: 0165-5728, DOI: 10.1016/S0165-5728(96)00167-1
- ALBERT RIES ET AL: "A novel biological function for CD44 in axon growth of retinal ganglion cells identified by a bioinformatics approach", JOURNAL OF NEUROCHEMISTRY, vol. 103, no. 4, 1 November 2007 (2007-11-01), pages 1491-1505, XP55035959, ISSN: 0022-3042, DOI: 10.1111/j.1471-4159.2007.04858.x

## Description

### FIELD AND BACKGROUND OF THE INVENTION

The present invention, in some embodiments thereof, relates to neuroprotective peptide agents.

Neurodegenerative disorders such as Alzheimer's disease (AD), Parkinson's Diseases (PD), Amyotrophic Lateral Sclerosis (ALS) and Huntington's disease (HD), are adult onset, chronic, progressive and irreversible severely disabling diseases in which progressive loss of structure and function of neurons, including death of neurons are present.

Alzheimer's disease (AD) is the most prevalent neurodegenerative disorder characterized by progressive loss of cognitive function. AD histopathology is defined by protein abnormalities namely plaques and neurofibrillary tangles which result from deposition of amyloid-β (Aβ) and hyperphosphorylated tau, respectively. These pathologies are accompanied by loss of neurons and white matter, congophilic angiopathy, inflammation and oxidative damage [1]. The role of inflammation in AD is evidenced by changes in microglia morphology and astrogliosis surrounding the senile plaque [2]. Aβ peptides are produced from the β-amyloid precursor protein (APP) through an initial β-secretase cleavage followed by the intramembraneous digestion by γ-secretase, a protein complex with presenilin1 at its catalytic core. The resulting peptide is secreted and deposited in the AD-defining amyloid plaques [1].

Parkinson's disease (PD) is a chronic and progressive neurodegenerative disease caused by a selective degeneration of dopaminergic neurons in the substantia nigra pars compacta of the brain. Symptoms include motor-related, including tremor, rigidity, slowness of movement, and postural instability. Among non-motor symptoms are autonomic dysfunction and sensory and sleep difficulties. Cognitive and neurobehavioral problems, including dementia, are common in the advanced stages of the disease. PD usually appears around the age of 60, although there are young-onset cases. The main pathological characteristic of PD is cell death in the substantia nigra and more specifically the ventral part of the pars compacta, affecting up to 70% of the cells by the time the patient dies [3]

CD44 codes for a family of class I transmembrane proteins which result from extensive alternative splicing and post translation modification. The variations are located in the extracellular membrane-proximal portion of the protein and are encoded by variants exons V2 (V1 in mice) to V10 [4] CD44 is the major cell surface receptor for hyaluronic acid (HA) but it has also been shown to bind proteins such as collagens, fibronectin, fibrinogen, laminin and osteopontin [5]. CD44 is essential for recruitment of circulating lymphocytes to the site of inflammation [6, 7]. CD44S, which doesn't contain any variant exon, is the most ubiquitous form and is expressed by most cell types [8]. CD44 variant proteins, in which one or more of the 10 variant exons are included, are mostly reported in association with cancer [9] and autoimmune diseases such as rheumatoid arthritis [10] and multiple sclerosis [11]. One of the unique functions suggested for CD44 splice variants is participation in signal transduction. As an example it was shown that CD44V6 is essential for signaling through tyrosine kinases such as c-Met [12] and VEGFR-2 [13].

In the brain CD44 is found predominantly in astrocytes of the white matter [14-18]. In contrast CD44 variants containing exons V4, V5 and V10 were localized to neurons [17]. CD44 expression was also found in activated microglial cells in the hippocampus following transient forebrain ischemia [19]. CD44 was first mentioned in association with AD when Akiyama et al reported a specific subset of CD44 positive astrocytes which number is increased dramatically in AD brains [14]. CD44 potential role in CNS regeneration was reported as it was found to be essential for axon growth of retinal ganglion cells [20]. CD44 was shown to play a role in ischemic brain injury as CD44-deficient mice had reduced infarct size compared with that of wild-type mice following middle cerebral artery occlusion [21]. Lammich et al [22] reported that CD44 goes through dual intramembraneous cleavage by a presenilin-dependent secretase [22] that liberates the extracellular domain as well as CD44 intracellular domain for putative nuclear signaling.

WO2009007934 teaches that that the expression of splice variants CD44V3, CD44V6 and CD44V10 are significantly increased in the hippocampi of AD patients compared to non-AD individuals

### SUMMARY OF THE INVENTION

The invention is directed to an isolated peptide and a pharmaceutical composition as defined in the claims.

According to an aspect of some embodiments of the present disclosure there is provided a method of treating a neurodegenerative disorder in a subject in need thereof, comprising administering to the subject a therapeutically effective amount of an isolated peptide comprising at least 3 amino acids of a CD44V10 amino acid sequence and no more than 100 amino acids of the CD44V10 amino acid sequence and comprising a neuroprotective activity, thereby treating the neurodegenerative disorder.

According to an aspect of some embodiments of the present disclosure there is provided a method of treating a neurodegenerative disorder in a subject in need thereof, comprising administering to the subject a therapeutically effective amount of an isolated peptide comprising at least 3 amino acids of a CD44V6 amino acid sequence and no more than 100 amino acids of the CD44V6 amino acid sequence and comprising a neuroprotective activity, thereby treating the neurodegenerative disorder.

According to an aspect of some embodiments of the present disclosure there is provided an isolated peptide comprising at least 3 amino acids of a CD44V10 amino acid sequence and no more than 20 amino acids of the CD44V10 amino acid sequence, with the proviso that the peptide does not consist of the amino acid sequence as set forth in SEQ ID NOs: 49 or 50, the peptide comprising a neuroprotective activity.

According to an aspect of some embodiments of the present disclosure there is provided an isolated peptide comprising at least 3 amino acids of a CD44V6 amino acid sequence and no more than 20 amino acids of the CD44V6 amino acid sequence, the peptide comprising a neuroprotective activity, with the proviso that the peptide does not consist of the amino acid sequence as set forth in SEQ ID NO: 1, 51 or 52.

According to an aspect of some embodiments of the present disclosure there is provided a pharmaceutical composition comprising as an active agent an isolated peptide comprising at least 3 amino acids of a CD44V10 amino acid sequence and no more than 100 amino acids of a CD44V10 amino acid sequence and comprising a neuroprotective activity and a pharmaceutically effective carrier.

According to an aspect of some embodiments of the present disclosure there is provided an isolated peptide comprising at least 3 amino acids of a CD44V10 amino acid sequence and no more than 100 amino acids of the CD44V10 amino acid sequence, and comprising a neuroprotective activity, for use in treating a neurodegenerative disorder.

According to some embodiments of the disclosure, the peptide comprises an amino acid sequence of formula 1:

X₁-G-Y-T-S,

wherein X₁ is any of a glutamic acid or glutamine.

According to some embodiments of the disclosure, the amino acid sequence comprises peptidomimetics.

According to some embodiments of the disclosure, the peptidomimetics comprises a retro-inverso mimetic.

According to some embodiments of the disclosure, the peptide is as set forth in SEQ ID NO: 26, 45 or 46.

According to some embodiments of the disclosure, the peptide consists of a CD44V10 amino acid sequence.

According to some embodiments of the disclosure, the CD44V10 amino acid sequence is a human CD44V10 amino acid sequence.

According to some embodiments of the disclosure, the CD44V6 amino acid sequence is a human CD44V6 amino acid sequence.

According to some embodiments of the disclosure, the peptide comprises a core sequence X₁-X₂-S-H, wherein X₁ and X₂ are acidic amino acids.

According to some embodiments of the disclosure, X₁ comprises glutamic acid.

According to some embodiments of the disclosure, X₂ comprises aspartic acid.

According to some embodiments of the disclosure, the peptide consists of a CD44V6 amino acid sequence.

According to some embodiments of the disclosure, the peptide comprises an amino acid sequence as set forth in SEQ ID NOs: 8-15, 18-45 or 46.

According to some embodiments of the disclosure, the peptide comprises an amino acid sequence as set forth in SEQ ID NOs: 49 or 50.

According to some embodiments of the disclosure, the peptide comprises an amino acid sequence as set forth in SEQ ID NOs: 2-7, 16 or 17.

According to an aspect of some embodiments of the present disclosure there is provided an isolated peptide comprising at least 3 amino acids of a CD44V6 amino acid sequence and no more than 100 amino acids of the CD44V6 amino acid sequence, and comprising a neuroprotective activity, for use in treating a neurodegenerative disorder.

According to some embodiments of the disclosure, the neurodegenerative disorder is selected from the group consisting of Parkinson's disease, Multiple Sclerosis, ALS, multi-system atrophy, Alzheimer's disease, stroke, traumatic brain injury, progressive supranuclear palsy, fronto-temporal dementia with parkinsonism linked to chromosome 17 and Pick's disease.

According to some embodiments of the disclosure, the neurodegenerative disease is Parkinson's disease.

According to some embodiments of the disclosure, the neurodegenerative disease is Alzheimer's disease.

According to some embodiments of the disclosure, the peptide comprises an amino acid sequence selected from the group consisting of SEQ ID NOs: 12, 15, 17, 19, 24, 26, 31, 32, 34, 36-38, 43-46.

According to some embodiments of the disclosure, the peptide comprises an amino acid sequence as set forth in SEQ ID NOs: 26 or 45.

According to an aspect of some embodiments of the present disclosure there is provided a pharmaceutical composition comprising as an active agent an isolated peptide comprising at least 3 amino acids of a CD44V6 amino acid sequence and no more than 100 amino acids of a CD44V6 amino acid sequence and comprising a neuroprotective activity and a pharmaceutically effective carrier.

According to some embodiments of the disclosure, the peptide is attached to a cell penetrating agent.

According to some embodiments of the disclosure, at least one of the amino acids is a naturally occurring amino acid.

According to some embodiments of the disclosure, at least one of the amino acids is a synthetic amino acid.

According to some embodiments of the disclosure, the synthetic amino acid comprises a D isomer.

According to some embodiments of the disclosure, the isolated peptide is covalently attached to the cell penetrating agent.

According to some embodiments of the disclosure, the cell penetrating agent is a peptide agent.

According to some embodiments of the disclosure, the peptide is no longer than 20 amino acids.

According to some embodiments of the disclosure, the peptide is 5-10 amino acids in length.

According to an aspect of some embodiments of the present disclosure there is provided a method of selecting an agent useful for treating a neurodegenerative disease, the method comprising:
(a) contacting a CD44v10/6 peptide with neuronal cells in the presence of a neurotoxic agent; and
(b) monitoring cell death of the neuronal cells, wherein a decrease in an amount or time of cell death of the neuronal cells in the presence of the CD44v10/6 peptide compared to an amount or time of cell death of the neuronal cells in the absence of the CD44v10/6 peptide is indicative of an agent useful for treating a neurodegenerative disease.

According to some embodiments of the disclosure, the neurotoxic agent is selected from the group consisting of an amyloid peptide, a glutamate, 6-OHDA, MPTP AND MPP+.

According to some embodiments of the disclosure, the administering comprises subcutaneous administering.

According to some embodiments of the disclosure, the administering comprises intranasal administering.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

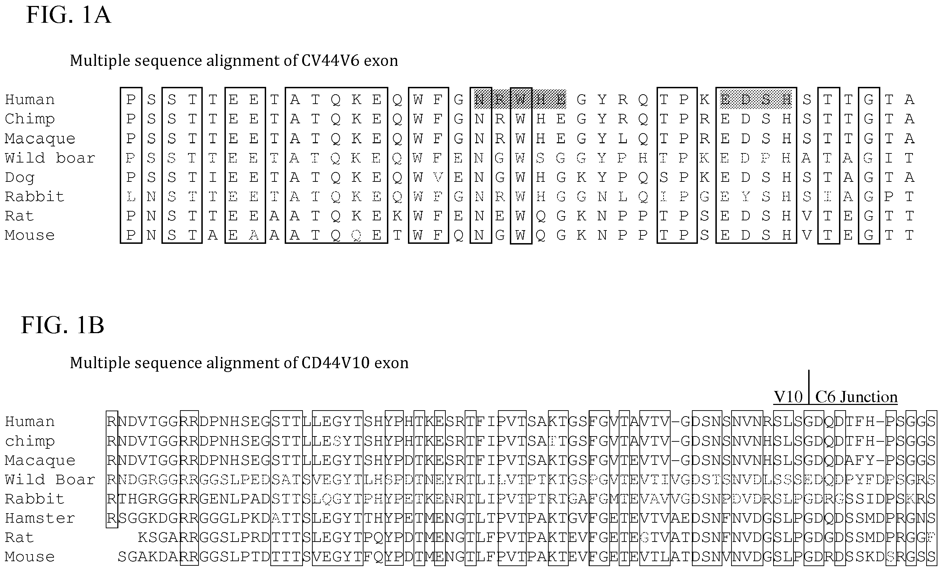

FIGs. 1A-B are multiple protein sequence alignments of CD44V6 (FIG. 1A) and CD44V10 (FIG. 1B) featuring the protein sequences of various mammalian organisms. The alignment was done using protein Blast algorithm online (NCBI). Conserved residues in which there is up to one non-conserved replacement in any of the species are marked by boxes. Non-conserved replacement in conserved residues are marked by red letter.

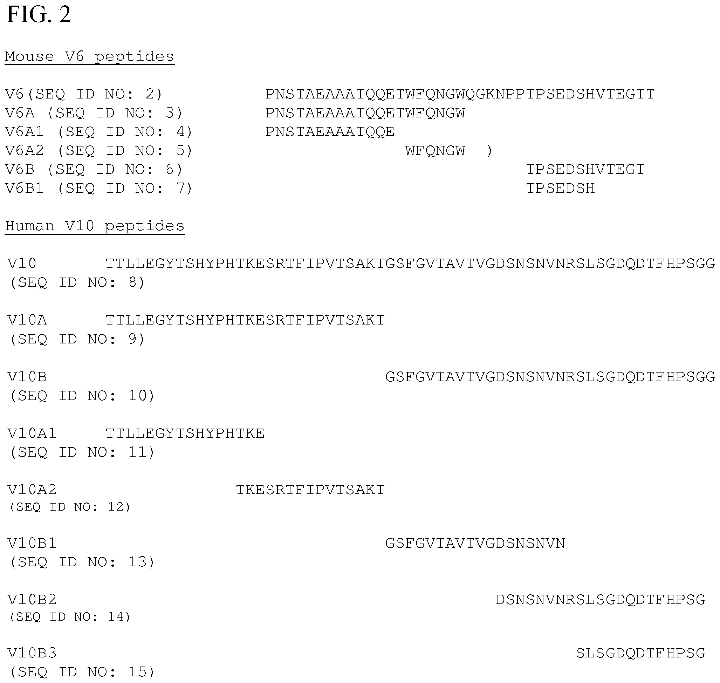

FIG. 2 shows a list of V6 (mouse) and V10 (human) peptides that their synthesis was guided according to the conserved regions denoted in FIG. 1.

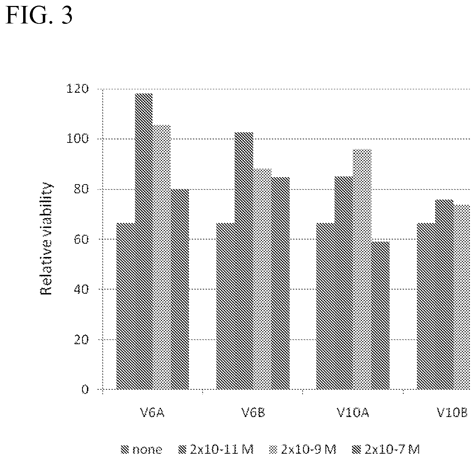

FIG. 3: is a bar graph showing the effect of V6 and V10 peptides at 3 concentrations on the viability of SK-N-SH human neuroblastoma cells, following treatment with 80 µM Aβ (1-42) for 48 hrs. Cellular viability was determined using the XTT colorimetric assay.

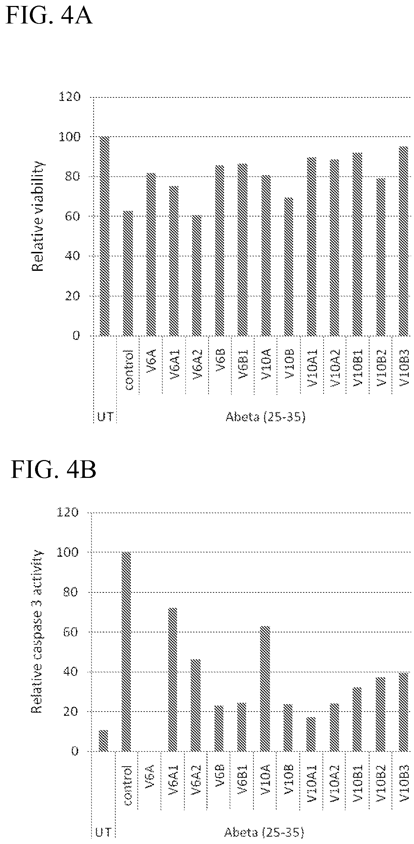

FIGs. 4A-B are bar graphs showing the effect of V6 and V10 peptides at 1 nM on the viability of N2A mouse neuroblastoma cells, following treatment with 25 µM Aβ (25-35) for 48 hrs. (FIG. 4A) Relative cellular viability as measured by XTT and (FIG. 4B) relative caspase 3 activity level are shown.

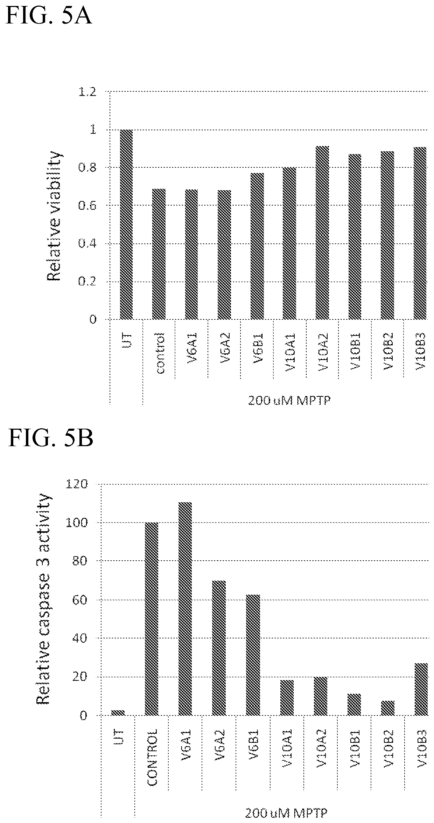

FIGs. 5A-B are bar graphs showing the effect of V6 and V10 peptides at 1 µM on the viability of N2A mouse neuroblastoma cells, following treatment with 200 µM MPTP for 48 hrs. Relative cellular viability (FIG. 5A) and caspase 3 activity level (FIG. 5B) are shown.

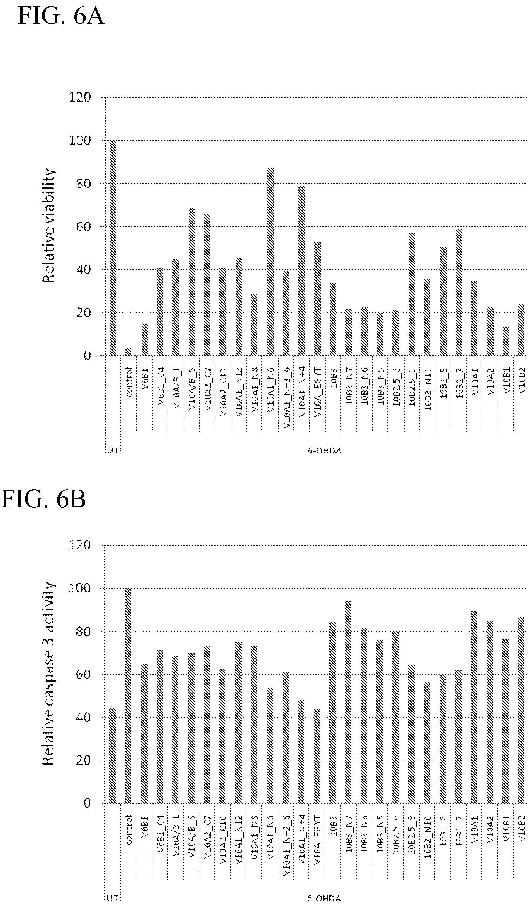

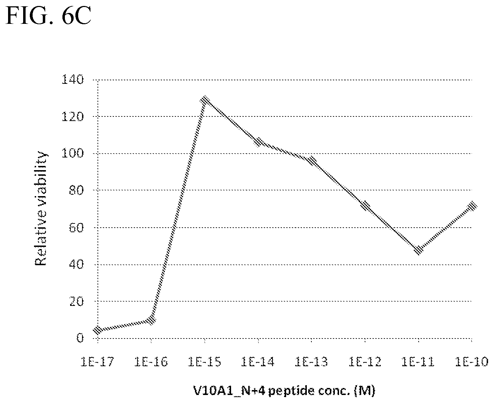

FIGs. 6A-C are graphs showing the effect of human V6 and V10 peptides that are listed in Table 1 and FIG. 2 at 1 pM on the viability of N2A mouse neuroblastoma cells, following treatment with 30 µM for 48 hrs. Relative viability (FIG. 6A) and caspase 3 activity (FIG. 6B) are shown. (FIG. 6C) The protective effect of N-acetylated and C-amidates V10A1_N+4 peptide against 30 µM 6-OHDA in N2A cells was tested at various concentration. Shown is the relative viability as measured by alamar blue fluorescence.

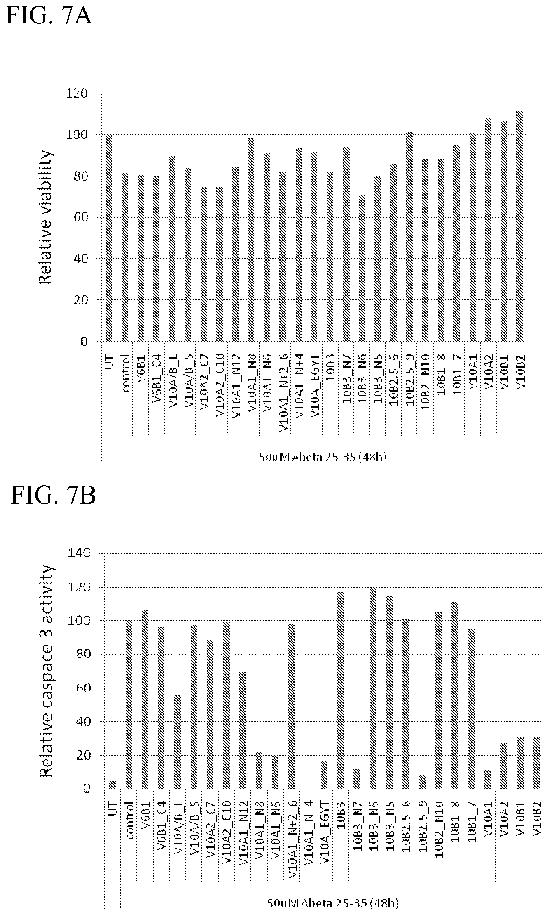

FIGs. 7A-B are bar graphs showing the effect of human V6 and V10 peptides that are listed in Table 1 and FIG. 2 at 1 pM on the viability of SK-N-SH human neuroblastoma cells, following treatment with 25 µM Aβ (25-35) for 48 hrs. Relative viability as measured by alamar blue fluorescence (FIG. 7A) and caspase 3 activity (FIG. 7B) are shown.

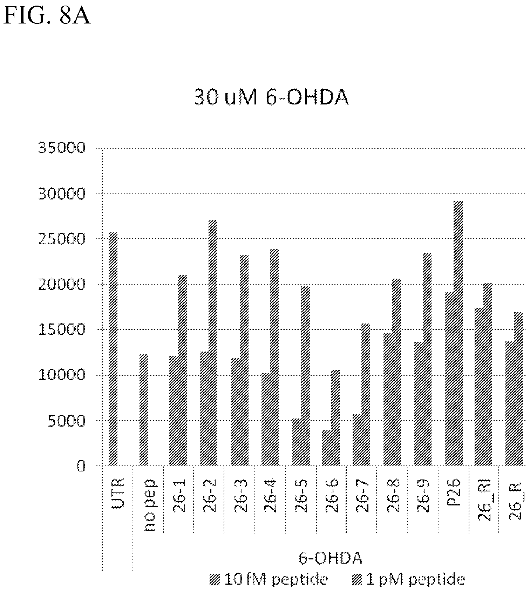

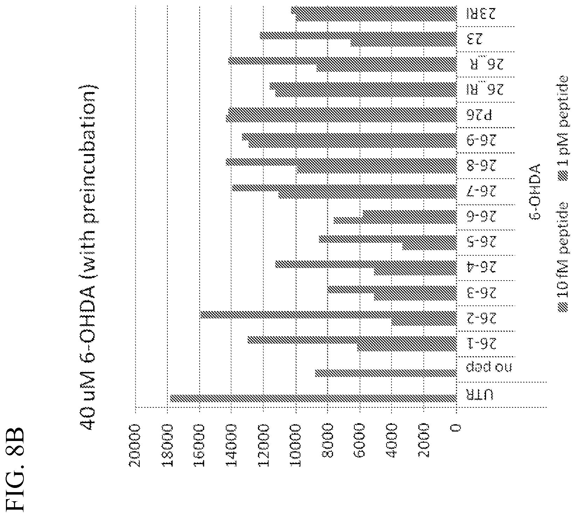

FIGs. 8A-B is a bar graph showing the effect of human P26-derived peptides that are listed in Table 3 at 10 fM and 1 pM on the viability of N2A mouse neuroblastoma cells, following treatment with 30 µM 6-OHDA (FIG. 8A) or 40µM (FIG. 8B) for 22 hrs. In FIG. 8B the cells were pre-incubated with the peptides for 2.3 hrs prior to the addition of 6-OHDA while in FIG. 8A, 6-OHDA was added together with the peptides. Relative viability as measured by alamar blue fluorescence is shown.

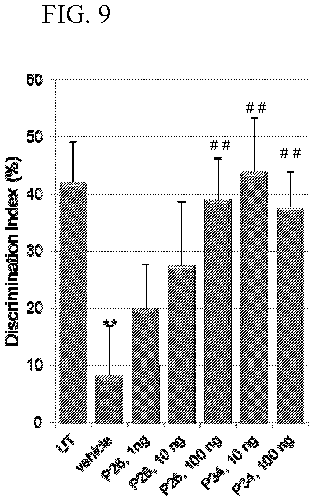

FIG. 9 is a bar graph showing the effects of repeated IH/ICV administration of P26 (SEQ ID NO: 26, 1, 10 and 100 ng/rat) or P34 (SEQ ID NO: 34 , 10 and 100 ng/rat) on the discrimination index on the retention test phase in the novel object recognition (NOR) task. ***P*<0.01 Aβ₍₁₋₄₂₎ with vehicle versus the control group (no Aβ₍₁₋₄₂₎) and ^{##}*P*<0.01 versus the vehicle control.

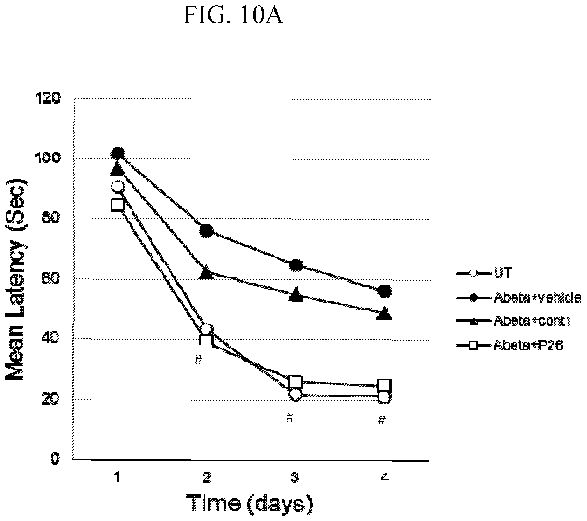

FIGs. 10A-B are graphs showing the effect of subcutaneous (SC) administered peptides on Morris wate maze (MWM) spatial memory assay following Aβ microinjection. A., The mean latency across 4 training sessions in MWM. Among the peptides-treated groups, the P26 group showed lower levels of the mean latency compared with the vehicle group (^{#}*p*<0.001 in days 2,3 and 4). B. shows the time spent in the target quadrant on test session. The P26 group showed higher levels of the time spent in the target quadrant compared with the vehicle group (^{#}*p<*0.05). UT - untreated group

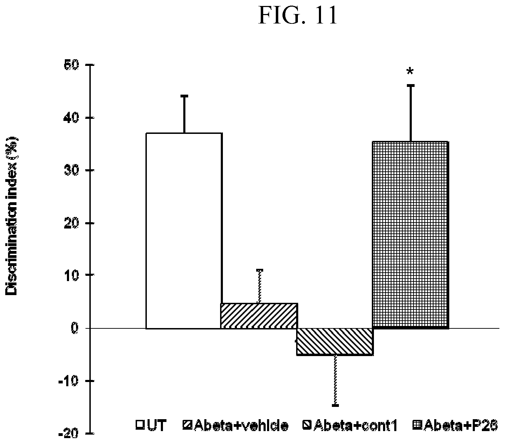

FIG. 11 is a bar graph showing the effect of SC administered peptides on NOR assay following Aβ microinjection. The discrimination index on the retention test phase is shown. **P*<0.05 versus the control group and ^{#}*p<*0.05 versus the Aβ only group

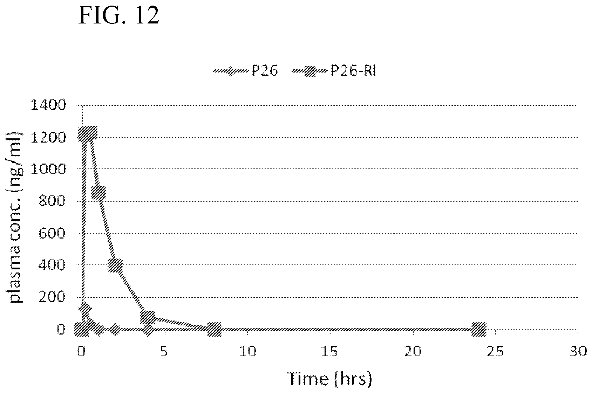

FIG. 12 is a graph showing the pharmacokinetics of P26 (SEQ ID NO: 26) and P26-RI (SEQ ID NO: 45) that was evaluated following subcutaneous administration of peptide solution in male Sprague Dawley rats at a dose of 1 mg/kg. LC-MS/MS method was used for the quantification of both peptides in plasma samples. The lower limit of quantification (LLOQ) was 22.34 ng/mL.

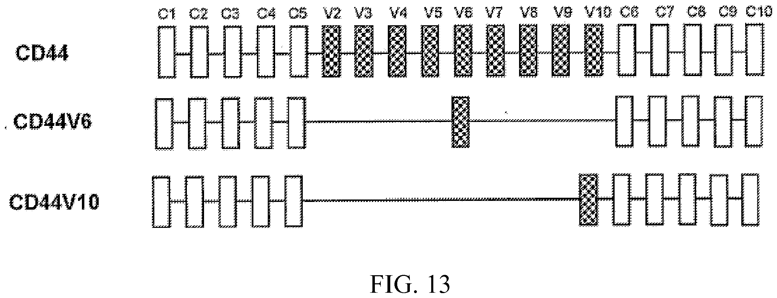

FIG. 13 is a schematic illustration of the genomic structure of CD44.

### DESCRIPTION OF SPECIFIC EMBODIMENTS OF THE INVENTION

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

It was previously found that the expression of splice variants CD44V3, CD44V6 and CD44V10 are significantly increased in the hippocampi of AD patients compared to non-AD individuals. The expression of the CD44 variants was further characterized and found to be mainly neuronal [23].

The present inventors characterized the function of multiple peptides derived from CD44 V6 and V10 exons sequences and found these peptides confer resistance to neuronal cells from neurotoxins such as beta amyloid (Aβ), MPTP and 6-OHDA, suggesting that these peptides or derivatives may serve as drugs for the treatment of neurodegenerative disorders.

The present inventors have performed structural-functional analyses to uncover minimal active domains that confer neuroprotection. The results were further substantiated in animal models for Pakinson's disease and Alzheimer's disease. These findings place the peptides of the instant invention as lead compounds for drug development.

Thus, there is disclosed an isolated peptide comprising at least 3 amino acids of a CD44V10 amino acid sequence and no more than 20 amino acids of said CD44V10 amino acid sequence wherein the peptide comprises a neuroprotective activity.

Also disclosed is an isolated peptide comprising at least 3 amino acids of a CD44V6 amino acid sequence and no more than 20 amino acids of said CD44V6 amino acid sequence, the peptide comprising a neuroprotective activity.

According to one embodiment the CD44V10 amino acid sequence does not consist of the sequence:
DSTDRIPATIRNDVTGGRR (SEQ ID NO: 49); or
NSNVNRSLSGDQDTFHPSG (SEQ ID NO: 50).

According to another embodiment the CD44 V6 amino acid sequence does not consist of the sequence:
DSTDRIPATIQATPSSTTE (SEQ ID NO: 51); or
DSHSTTGTAGDQDTFHPSG (SEQ ID NO: 52).

Peptides comprising the amino acid sequence set forth in SEQ ID NOs: 49-52 are contemplated for use in the treatment of neurodegenerative diseases, as further elaborated hereinbelow.

As used herein "CD44" refers to the cell surface protein that is expressed in a large number of mammalian cell types as set forth in RefSeq Accession No: NM_000610.3. According to a specific embodiment the CD44 is the human CD44 gene. The standard isoform, designated CD44, comprising exons 1-5 and 16-20 is expressed in most cell types. The gene structure is provided in Figure 13 including that of the splice variants CD44V6 and CD44V10.

As used herein, the term " CD44V10" corresponds to amino acid coordinates 537-604 of SEQ ID NO: 53, RefSeq Accession No: NP_000601.3 (human CD44 antigen isoform 1 precursor, NCBI Reference Sequence) and is exemplified by SEQ ID NO:2.

As used herein, the term " CD44V6" corresponds to amino acid coordinates 386-427 of SEQ ID NO: 53, RefSeq Accession No: NP_000601.3 (human CD44 antigen isoform 1 precursor, NCBI Reference Sequence) and is exemplified by SEQ ID NO: 8.

As used herein, the phrase "neuroprotective activity" refers to prevention of neural cell death. The effect may take the form of protection of neuronal cells i.e., neurons, from apoptosis or degeneration. Assays for qualifying a neuroprotective activity include cell viability assays (e.g., XTT, MTT), morphological assays (e.g., cell staining) or apoptosis biochemical assays (e.g., caspase 3 activity and the like).

According to a specific embodiment, the CD44V6 amino acid sequence is a human CD44V6 amino acid sequence.

According to a specific embodiment, the CD44V10 amino acid sequence is a human CD44V10 amino acid sequence.

According to a further specific embodiment, the peptide consists of a CD44V6 amino acid sequence (SEQ ID NO: 2).

While further reducing the present invention to practice, the present inventors have uncovered that the peptidic portion (amino acid sequence) which imparts neuroprotection comprises a core sequence X₁-X₂-S-H, wherein X₁ and X₂ are acidic amino acids.

As used herein, the phrase "acidic amino acid" refers to naturally occurring or synthetic amino acids which are polar and negatively charged at physiological pH.

According to a specific embodiment, the X₁ comprises glutamic acid. According to a specific embodiment, the X₂ comprises aspartic acid.

According to a specific embodiment, the peptide comprises the amino acid sequence of SEQ ID NO: 6 or 7.

According to a further embodiment, the CD44V6 consists of a CD44V6 amino acid sequence (SEQ ID NO: 2).

According to a further embodiment, the peptide comprises an amino acid sequence as set forth in SEQ ID NOs: 2-7, 16 or 17.

As mentioned, peptides of CD44V10 are also contemplated herein. Thus, according to an exemplary embodiment the peptide comprises an amino acid sequence as set forth in SEQ ID NOs: 8-15, 18-46, or specifically, SEQ ID NO: 8-15, 18-38, 39-42 or 43-46.

While further reducing the present invention to practice, the present inventors were able to identify a minimal portion of CD44V10 which is active in conferring neuroprotection.

Thus, according to an exemplary embodiment, the CD44V10 peptide comprises an amino acid sequence of formula 1:

X₁-G-Y-T-S,

wherein X₁ is any of a glutamic acid or glutamine.

As will be further described in details hereinbelow, the amino acid sequence of the peptide comprises peptidomimetics, such as a retro-inverso mimetic (e.g., SEQ ID NO: 45 or 46).

According to an exemplary embodiment, the peptide is as set forth in SEQ ID NO: 26.

According to an exemplary embodiment, the peptide is as set forth in SEQ ID NO: 26, 45 or 46.

According to a further specific embodiment, the peptide consists of a CD44V10 amino acid sequence (SEQ ID NO: 8).

The term "peptide" as used herein refers to a polymer of natural or synthetic amino acids, encompassing native peptides (either degradation products, synthetically synthesized polypeptides or recombinant polypeptides) and peptidomimetics (e.g., inverso, retro or retro-inverso, typically, synthetically synthesized peptides), as well as peptoids and semipeptoids which are polypeptide analogs, which may have, for example, modifications rendering the peptides even more stable while in a body or more capable of penetrating into cells.

Such modifications include, but are not limited to N terminus modification, C terminus modification, polypeptide bond modification, including, but not limited to, CH2-NH, CH2-S, CH2-S=O, O=C-NH, CH2-O, CH2-CH2, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992). Further details in this respect are provided hereinunder.

Polypeptide bonds (-CO-NH-) within the polypeptide may be substituted, for example, by N-methylated bonds (-N(CH3)-CO-), ester bonds (-C(R)H-C-O-O-C(R)-N-), ketomethylen bonds (-CO-CH2-), α-aza bonds (-NH-N(R)-CO-), wherein R is any alkyl, e.g., methyl, carba bonds (-CH2-NH-), hydroxyethylene bonds (-CH(OH)-CH2-), thioamide bonds (-CS-NH-), olefinic double bonds (-CH=CH-), retro amide bonds (-NH-CO-), polypeptide derivatives (-N(R)-CH2-CO-), wherein R is the "normal" side chain, naturally presented on the carbon atom.

These modifications can occur at any of the bonds along the polypeptide chain and even at several (2-3) at the same time.

Natural aromatic amino acids, Trp, Tyr and Phe, may be substituted for synthetic non-natural acid such as Phenylglycine, naphthylelanine (Nol), ring-methylated derivatives of Phe, halogenated derivatives of Phe or o-methyl-Tyr.

In addition to the above, the polypeptides may also include one or more modified amino acids or one or more non-amino acid monomers (e.g. fatty acids, complex carbohydrates etc).

As used herein in the specification and in the claims section below the term "amino acid" or "amino acids" is understood to include the 20 naturally occurring amino acids; those amino acids often modified post-translationally *in vivo*, including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids (stereoisomers).

Tables A and B below list naturally occurring amino acids (Table A) and non-conventional or modified amino acids (Table B) which can be used with the present invention.

**Table A**

| **Amino Acid** | **Three**-**Letter Abbreviation** | **One-letter Symbol** |
|---|---|---|
| alanine | Ala | A |
| Arginine | Arg | R |
| Asparagine | Asn | N |
| Aspartic acid | Asp | D |
| Cysteine | Cys | C |
| Glutamine | Gln | Q |
| Glutamic Acid | Glu | E |
| glycine | Gly | G |
| Histidine | His | H |
| isoleucine | lie | I |
| leucine | Leu | L |
| Lysine | Lys | K |
| Methionine | Met | M |
| phenylalanine | Phe | F |
| Proline | Pro | P |
| Serine | Ser | S |
| Threonine | Thr | T |
| tryptophan | Trp | W |
| tyrosine | Tyr | Y |
| Valine | Val | V |
| Any amino acid as above | Xaa | X |

**Table B**

| **Non**-**conventional amino acid** | **Code** | **Non**-**conventional amino acid** | **Code** |
|---|---|---|---|
| α-aminobutyric acid | Abu | L-N-methylalanine | Nmala |
| α -amino- α-methylbutyrate | Mgabu | L-N-methylarginine | Nmarg |
| aminocyclopropane- | Cpro | L-N-methylasparagine | Nmasn |
| carboxylate | | L-N-methylaspartic acid | Nmasp |
| aminoisobutyric acid | Aib | L-N-methylcysteine | Nmcys |
| aminonorbornyl- | Norb | L-N-methylglutamine | Nmgin |
| carboxylate | | L-N-methylglutamic acid | Nmglu |
| cyclohexylalanine | Chexa | L-N-methylhistidine | Nmhis |
| cyclopentylalanine | Cpen | L-N-methylisolleucine | Nmile |
| D-alanine | Dal | L-N-methylleucine | Nmleu |
| D-arginine | Darg | L-N-methyllysine | Nmlys |
| D-aspartic acid | Dasp | L-N-methylmethionine | Nmmet |
| D-cysteine | Dcys | L-N-methylnorleucine | Nmnle |
| D-glutamine | Dgln | L-N-methylnorvaline | Nmnva |
| D-glutamic acid | Dglu | L-N-methylornithine | Nmorn |
| D-histidine | Dhis | L-N-methylphenylalanine | Nmphe |
| D-isoleucine | Dile | L-N-methylproline | Nmpro |
| D-leucine | Dleu | L-N-methylserine | Nmser |
| D-lysine | Dlys | L-N-methylthreonine | Nmthr |
| D-methionine | Dmet | L-N-methyltryptophan | Nmtrp |
| D-ornithine | Dorn | L-N-methyltyrosine | Nmtyr |
| D-phenylalanine | Dphe | L-N-methylvaline | Nmval |
| D-proline | Dpro | L-N-methylethylglycine | Nmetg |
| D-serine | Dser | L-N-methyl-t-butylglycine | Nmtbug |
| D-threonine | Dthr | L-norleucine | Nle |
| D-tryptophan | Dtrp | L-norvaline | Nva |
| D-tyrosine | Dtyr | α -methyl-aminoisobutyrate | Maib |
| D-valine | Dval | α -methyl-γ-aminobutyrate | Mgabu |
| D- α -methylalanine | Dmala | α ethylcyclohexylalanine | Mchexa |
| D- α -methylarginine | Dmarg | α-methylcyclopentylalanine | Mcpen |
| D- α-methylasparagine | Dmasn | α -methyl- α-napthylalanine | Manap |
| D- α -methylaspartate | Dmasp | α-methylpenicillamine | Mpen |
| D- α -methylcysteine | Dmcys | N-(4-aminobutyl) glycine | Nglu |
| D- α-methylglutamine | Dmgln | N-(2-aminoethyl) glycine | Naeg |
| D- α -methylhistidine | Dmhis | N-(3-aminopropyl)glycine | Norn |
| D- α-methylisoleucine | Dmile | N- amino- α-methylbutyrate | Nmaabu |
| D- α -methylleucine | Dmleu | α -napthylalanine | Anap |
| D- α -methyllysine | Dmlys | N-benzylglycine | Nphe |
| D- α-methylmethionine | Dmmet | N-(2-carbamylethyl)glycine | Ngln |
| D- α -methylornithine | Dmorn | N-(carbamylmethyl)glycine | Nasn |
| D- α-methylphenylalanine | Dmphe | N-(2-carboxyethyl)glycine | Nglu |
| D- α -methylproline | Dmpro | N-(carboxymethyl)glycine | Nasp |
| D- α -methylserine | Dmser | N-cyclobutylglycine | Ncbut |
| D- α -methylthreonine | Dmthr | N-cycloheptylglycine | Nchep |
| D- α-methyltryptophan | Dmtrp | N-cyclohexylglycine | Nchex |
| D- α -methyltyrosine | Dmty | N-cyclodecylglycine | Ncdec |
| D- α -methylvaline | Dmval | N-cyclododeclglycine | Ncdod |
| D- α -methylalnine | Dnmala | N-cyclooctylglycine | Ncoct |
| D- α -methylarginine | Dnmarg | N-cyclopropylglycine | Ncpro |
| D- α-methylasparagine | Dnmasn | N-cycloundecylglycine | Ncund |
| D- α-methylasparatate | Dnmasp | N-(2,2-diphenylethyl) glycine | Nbhm |
| D- α -methylcysteine | Dnmcys | N-(3,3-diphenylpropyl)glycine | Nbhe |
| D-N-methylleucine | Dnmleu | N-(3-indolylyethyl) glycine | Nhtrp |
| D-N-methyllysine | Dnmlys | N-methyl-7-aminobutyrate | Nmgabu |
| N-methylcyclohexylalanine | Nmchex | D-N-methylmethionine | Dnmme |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl) glycine | Nva |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gaba | N-(*p-*hydroxyphenyl) glycine | Nhtyr |
| L-*t*-butylglycine | Tbug | N-(thiomethyl) glycine | Ncys |
| L-ethylglycine | Etg | penicillamine | Pen |
| L-homophenylalanine | Hphe | L- α -methylalanine | Mala |
| L- α-methylarginine | Marg | L- α-methylasparagine | Masn |
| L- α-methylaspartate | Masp | L- α -methyl-t-butylglycine | Mtbug |
| L- α-methylcysteine | Mcys | L-methylethylglycine | Metg |
| L- α thylglutamine | Mgln | L- α-methylglutamate | Mglu |
| L- α-methylhistidine | Mhis | L- α -methylhomo phenylalanine | Mhphe |
| L- α-methylisoleucine | Mile | N-(2-methylthioethyl)glycine | Nmet |
| D-N-methylglutamine | Dnmgln | N-(3-guanidinopropyl)glycine | Narg |
| D-N-methylglutamate | Dnmglu | N-(1-hydroxyethyl)glycine | Nthr |
| D-N-methylhistidine | Dnmhis | N-(hydroxyethyl)glycine | Nser |
| D-N-methylisoleucine | Dnmile | N-(imidazolylethyl)glycine | Nhis |
| D-N-methylleucine | Dnmleu | N-(3-indolylyethyl)glycine | Nhtrp |
| D-N-methyllysine | Dnmlys | N-methyl-7-aminobutyrate | Nmgabu |
| N-methylcyclohexylalanine | Nmchex | D-N-methylmethionine | Dnmme |
| D-N-methylornithine | Dnmorn | N-methylcyclopentylalanine | Nmcpen |
| N-methylglycine | Nala | D-N-methylphenylalanine | Dnmphe |
| N-methylaminoisobutyrate | Nmaib | D-N-methylproline | Dnmpro |
| N-(1-methylpropyl)glycine | Nile | D-N-methylserine | Dnmser |
| N-(2-methylpropyl)glycine | Nleu | D-N-methylthreonine | Dnmthr |
| D-N-methyltryptophan | Dnmtrp | N-(1-methylethyl) glycine | Nval |
| D-N-methyltyrosine | Dnmtyr | N-methyla-napthylalanine | Nmanap |
| D-N-methylvaline | Dnmval | N-methylpenicillamine | Nmpen |
| γ-aminobutyric acid | Gabu | N-(*p-*hydroxyphenyl) glycine | Nhtyr |
| L-*t*-butylglycine | Tbug | N-(thiomethyl) glycine | Ncys |
| L-ethylglycine | Etg | penicillamine | Pen |
| L-homophenylalanine | Hphe | L- α -methylalanine | Mala |
| L- α-methylarginine | Marg | L- α-methylasparagine | Masn |
| L- α-methylaspartate | Masp | L- α -methyl-t-butylglycine | Mtbug |
| L- α-methylcysteine | Mcys | L-methylethylglycine | Metg |
| L- α-methylglutamine | Mgln | L- α-methylglutamate | Mglu |
| L- α ethylhistidine | Mhis | L- α-methylhomophenylalanine | Mhphe |
| L- α thylisoleucine | Mile | N-(2-methylthioethyl)glycine | Nmet |
| L- α -methylleucine | Mleu | L- α -methyllysine | Mlys |
| L- α-methylmethionine | Mmet | L- α-methylnorleucine | Mnle |
| L- α-methylnorvaline | Mnva | L- α-methylornithine | Morn |
| L- α-methylphenylalanine | Mphe | L- α -methylproline | Mpro |
| L- α -methylserine | mser | L- α-methylthreonine | Mthr |
| L- α ethylvaline | Mtrp | L- α -methyltyrosine | Mtyr |
| L- α -methylleucine | Mval Nnbhm | L-N-methylhomophenylalanine | Nmhphe |
| N-(N-(2,2-diphenylethyl) | | N-(N-(3,3-diphenylpropyl) | |
| carbamylmethyl-glycine | Nnbhm | carbamylmethyl(1)gl ycine | Nnbhe |
| 1-carboxy-1-(2,2-diphenyl ethylamino)cyclopropane | Nmbc | | |

The amino acids of the peptides of the present invention may be substituted either conservatively or non-conservatively.

The term "conservative substitution" as used herein, refers to the replacement of an amino acid present in the native sequence in the peptide with a naturally or non-naturally occurring amino or a peptidomimetics having similar steric properties. Where the side-chain of the native amino acid to be replaced is either polar or hydrophobic, the conservative substitution should be with a naturally occurring amino acid, a non-naturally occurring amino acid or with a peptidomimetic moiety which is also polar or hydrophobic (in addition to having the same steric properties as the side-chain of the replaced amino acid).

As naturally occurring amino acids are typically grouped according to their properties, conservative substitutions by naturally occurring amino acids can be easily determined bearing in mind the fact that in accordance with the invention replacement of charged amino acids by sterically similar non-charged amino acids are considered as conservative substitutions.

For producing conservative substitutions by non-naturally occurring amino acids it is also possible to use amino acid analogs (synthetic amino acids) well known in the art. A peptidomimetic of the naturally occurring amino acid is well documented in the literature known to the skilled practitioner.

When affecting conservative substitutions the substituting amino acid should have the same or a similar functional group in the side chain as the original amino acid.

The phrase "non-conservative substitutions" as used herein refers to replacement of the amino acid as present in the parent sequence by another naturally or non-naturally occurring amino acid, having different electrochemical and/or steric properties. Thus, the side chain of the substituting amino acid can be significantly larger (or smaller) than the side chain of the native amino acid being substituted and/or can have functional groups with significantly different electronic properties than the amino acid being substituted. Examples of non-conservative substitutions of this type include the substitution of phenylalanine or cycohexylmethyl glycine for alanine, isoleucine for glycine, or -NH-CH[(-CH₂)₅₋COOH]-CO- for aspartic acid. Those non-conservative substitutions which fall under the scope of the present invention are those which still constitute a peptide having neuroprotective properties.

As mentioned, the N and C termini of the peptides of the present invention may be protected by function groups. Suitable functional groups are described in Green and Wuts, "Protecting Groups in Organic Synthesis", John Wiley and Sons, Chapters 5 and 7, 1991. Preferred protecting groups are those that facilitate transport of the compound attached thereto into a cell, for example, by reducing the hydrophilicity and increasing the lipophilicity of the compounds.

These moieties can be cleaved *in vivo*, either by hydrolysis or enzymatically, inside the cell. Hydroxyl protecting groups include esters, carbonates and carbamate protecting groups. Amine protecting groups include alkoxy and aryloxy carbonyl groups, as described above for N-terminal protecting groups. Carboxylic acid protecting groups include aliphatic, benzylic and aryl esters, as described above for C-terminal protecting groups. In one embodiment, the carboxylic acid group in the side chain of one or more glutamic acid or aspartic acid residue in a peptide of the present invention is protected, preferably with a methyl, ethyl, benzyl or substituted benzyl ester.

Examples of N-terminal protecting groups include acyl groups (-CO-R1) and alkoxy carbonyl or aryloxy carbonyl groups (-CO-O-R1), wherein R1 is an aliphatic, substituted aliphatic, benzyl, substituted benzyl, aromatic or a substituted aromatic group. Specific examples of acyl groups include acetyl, (ethyl)-CO-, n-propyl-CO-, iso-propyl-CO-, n-butyl-CO-, sec-butyl-CO-, t-butyl-CO-, hexyl, lauroyl, palmitoyl, myristoyl, stearyl, oleoyl phenyl-CO-, substituted phenyl-CO-, benzyl-CO- and (substituted benzyl)-CO-. Examples of alkoxy carbonyl and aryloxy carbonyl groups include CH3-O-CO-, (ethyl)-O-CO-, n-propyl-O-CO-, iso-propyl-O-CO-, n-butyl-O-CO-, sec-butyl-O-CO-, t-butyl-O-CO-, phenyl-O- CO-, substituted phenyl-O-CO- and benzyl-O-CO-, (substituted benzyl)- O-CO-. Adamantan, naphtalen, myristoleyl, tuluen, biphenyl, cinnamoyl, nitrobenzoy, toluoyl, furoyl, benzoyl, cyclohexane, norbornane, Z-caproic. In order to facilitate the N-acylation, one to four glycine residues can be present in the N-terminus of the molecule.

The carboxyl group at the C-terminus of the compound can be protected, for example, by an amide (i.e., the hydroxyl group at the C-terminus is replaced with -NH₂, -NHR₂ and -NR₂R₃) or ester (i.e. the hydroxyl group at the C-terminus is replaced with -OR₂). R₂ and R₃ are independently an aliphatic, substituted aliphatic, benzyl, substituted benzyl, aryl or a substituted aryl group. In addition, taken together with the nitrogen atom, R₂ and R₃ can form a C4 to C8 heterocyclic ring with from about 0-2 additional heteroatoms such as nitrogen, oxygen or sulfur. Examples of suitable heterocyclic rings include piperidinyl, pyrrolidinyl, morpholino, thiomorpholino or piperazinyl. Examples of C-terminal protecting groups include -NH₂, -NHCH₃, -N(CH₃)₂, -NH(ethyl), -N(ethyl)₂, -N(methyl) (ethyl), -NH(benzyl), -N(C1-C4 alkyl)(benzyl), -NH(phenyl), -N(C1-C4 alkyl) (phenyl), -OCH₃, -O-(ethyl), -O-(n-propyl), -O-(n-butyl), -O-(iso-propyl), -O-(sec-butyl), -O-(t-butyl), -O-benzyl and -O-phenyl.

Of note, in this disclosure peptides (derived from either CD44V6, CD44V10, as described above) are referred to in general as CD44 peptides.

The CD44 peptides (i.e., the neuroprotecting peptide portion) is 3-100, or 3-50, or 3-40, or 3-30 amino acids in length. According to further embodiments, the peptide is 3-20, 5-20, 5-20, 5-18, 5-15, 5-10, 7-10, 8-10 amino acids in length.

The CD44 peptides may be qualified for their neuroprotective activity as described hereinabove and in the Examples section which follows using both in vitro and in vivo models for neuroprotection and neurodegenerative conditions.

The present teachings may further be employed for the identification of agents useful for treating a neurodegenerative disease.

Thus, there is disclosed a method comprising:
(a) contacting a CD44v10/6 peptide with neuronal cells in the presence of a neurotoxic agent; and
(b) monitoring cell death of said neuronal cells, wherein a decrease in an amount or time of cell death of said neuronal cells in the presence of said CD44v10/6 peptide compared to an amount or time of cell death of said neuronal cells in the absence of said CD44v10/6 peptide is indicative of an agent useful for treating a neurodegenerative disease.

Methods of monitoring neural cell death are well known in the art and are further described hereinabove (under neuroprotection) and in the Examples section which follows.

A neurotoxic agent as used herein refers to a molecule a condition or state that damages the nervous system and/or brain, usually by killing neurons.

According to a specific embodiment, the neurotoxic agent is selected from the group consisting of an amyloid, a glutamate, 6-OHDA, MPTP AND MPP+.

In order to improve the bioavailability of the CD44 peptides, a single, a portion or even all the amino acids in the peptide can be D amino acids which are not susceptible to enzymatic proteolytic activity and can improve altogether the use of the peptides of the invention as pharmaceuticals. The peptides of the present invention may be attached (either covalently or non-covalently) to a penetrating agent.

As used herein the phrase "penetrating agent" refers to an agent which enhances translocation of any of the attached peptide across a cell membrane.

According to one embodiment, the penetrating agent is a peptide and is attached to the CD44 peptides (either directly or non-directly) via a peptide bond.

Typically, peptide penetrating agents have an amino acid composition containing either a high relative abundance of positively charged amino acids such as lysine or arginine, or have sequences that contain an alternating pattern of polar/charged amino acids and non-polar, hydrophobic amino acids.

By way of non-limiting example , cell penetrating peptide (CPP) sequences may be used in order to enhance intracellular penetration. CPPs may include short and long versions of the protein transduction domain (PTD) of HIV TAT protein [YGRKKRR (SEQ ID NO: 54), YGRKKRRQRRR (SEQ ID NO: 55), or RRQRR (SEQ ID NO: 56)]. However, the disclosure is not so limited, and any suitable penetrating agent may be used, as known by those of skill in the art.

According to a particular embodiment, the peptide conjugates of the present invention are no longer than 25, 30 or 40 amino acids (this includes the CD44 peptide together with any additional attached sequence, such as a cell penetrating peptide as described above).

The peptides may also comprise non-amino acid moieties, such as for example, hydrophobic moieties (various linear, branched, cyclic, polycyclic or hetrocyclic hydrocarbons and hydrocarbon derivatives) attached to the peptides; non-peptide penetrating agents; various protecting groups, especially where the compound is linear, which are attached to the compound's terminals to decrease degradation. Chemical (non-amino acid) groups present in the compound may be included in order to improve various physiological properties such; decreased degradation or clearance; decreased repulsion by various cellular pumps, improve immunogenic activities, improve various modes of administration (such as attachment of various sequences which allow penetration through various barriers, through the gut, etc.); increased specificity, increased affinity, decreased toxicity and the like.

Attaching the amino acid sequence component of the peptides of the invention to other non-amino acid agents may be by covalent linking, by non-covalent complexion, for example, by complexion to a hydrophobic polymer, which can be degraded or cleaved producing a compound capable of sustained release; by entrapping the amino acid part of the peptide in liposomes or micelles to produce the final peptide. The association may be by the entrapment of the amino acid sequence within the other component (liposome, micelle) or the impregnation of the amino acid sequence within a polymer to produce the final peptide.

The peptides may be linear or cyclic (cyclization may improve stability). Cyclization may take place by any means known in the art. Where the compound is composed predominantly of amino acids, cyclization may be via N- to C-terminal, N-terminal to side chain and N-terminal to backbone, C-terminal to side chain, C-terminal to backbone, side chain to backbone and side chain to side chain, as well as backbone to backbone cyclization. Cyclization of the peptide may also take place through non-amino acid organic moieties comprised in the peptide.

The peptides of the present invention can be biochemically synthesized such as by using standard solid phase techniques. These methods include exclusive solid phase synthesis, partial solid phase synthesis methods, fragment condensation, classical solution synthesis. Solid phase polypeptide synthesis procedures are well known in the art and further described by John Morrow Stewart and Janis Dillaha Young, Solid Phase Polypeptide Syntheses (2nd Ed., Pierce Chemical Company, 1984).

Large scale peptide synthesis is described by Andersson Biopolymers 2000;55(3):227-50.

Synthetic peptides can be purified by preparative high performance liquid chromatography [Creighton T. (1983) Proteins, structures and molecular principles. WH Freeman and Co. N.Y.] and the composition of which can be confirmed via amino acid sequencing.

Recombinant techniques may also be used to generate the peptides of the present invention. To produce a peptide of the present invention using recombinant technology, a polynucleotide encoding the peptide of the present invention is ligated into a nucleic acid expression vector, which comprises the polynucleotide sequence under the transcriptional control of a cis-regulatory sequence (e.g., promoter sequence) suitable for directing constitutive, tissue specific or inducible transcription of the polypeptides of the present invention in the host cells.

In addition to being synthesizable in host cells, the peptides of the present invention can also be synthesized using *in vitro* expression systems. These methods are well known in the art and the components of the system are commercially available.

As mentioned, by virtue of their neuroprotective function, the peptides of the present invention may be used to treat neurodegenerative disorders.

Thus, there is disclosed a method of treating a neurodegenerative disorder in a subject in need thereof, comprising administering to the subject a therapeutically effective amount of an isolated peptide comprising at least 3 amino acids of a CD44V10 amino acid sequence and no more than 20 amino acids of said CD44V10 amino acid sequence and comprising a neuroprotective activity, thereby treating the neurodegenerative disorder.

According to another aspect, there is disclosed a method of treating a neurodegenerative disorder in a subject in need thereof, comprising administering to the subject a therapeutically effective amount of an isolated peptide comprising at least 3 amino acids of a CD44V6 amino acid sequence and no more than 20 amino acids of said CD44V6 amino acid sequence and comprising a neuroprotective activity, thereby treating the neurodegenerative disorder.

As used herein, the phrase "a subject in need thereof" or "a subject" refers to mammals, preferably human beings at any age which suffer from a neural damage or is at risk to develop a neural damage.

As used herein the phrase "neural damage" refers to any disease, disorder or condition which is characterized by an acute and/or progressive damage and/or loss of neuronal cells and/or glial cells.

According to some embodiments, the pathology associated with neural damage affects neuronal and/or glial cells in the central nervous system.

Non-limiting examples of pathologies caused by an acute or sudden damage to neuronal cells include brain injury, spinal injury, head injury, and stroke [cerebrovascular accident (CVA)].

According to some embodiments, the pathology associated with neural damage is cancer. Non-limiting examples of cancers which affect the neuronal and glial cells include glioblastoma, neuroblastoma, adenocarcinoma of the brain, as well as metastases of a distant cancers such as breast cancer, lung cancer, and the like.

According to some embodiments, the pathology associated with neural damage is chronic.

According to some embodiments, the pathology associated with neural damage is a neurodegenerative disease.

Exemplary neurodegenerative diseases or conditions include, but are not limited to multi-system atrophy, stroke, progressive supranuclear palsy, fronto-temporal dementia with parkinsonism linked to chromosome 17, traumatic brain injury (TBI), Pick's disease, multiple sclerosis, Lupus eruthromatosis, Alzheimer's disease, Parkinson's Disease, senile dementia, amyotrophic lateral sclerosis, Down's Syndrome, Dutch Type Hereditary Cerebral Hemorrhage Amyloidosis, Reactive Amyloidosis, Familial Mediterranean Fever, Familial Amyloid Nephropathy with Urticaria and Deafness, Muckle-Wells Syndrome, Idiopathic Myeloma, Macroglobulinemia-Associated Myeloma, Familial Amyloid Polyneuropathy, Familial Amyloid Cardiomyopathy, Isolated Cardiac Amyloid, Systemic Senile Amyloidosis, Adult Onset Diabetes, Insulinoma, Isolated Atrial Amyloid, Medullary Carcinoma of the Thyroid, Familial Amyloidosis, Hereditary Cerebral Hemorrhage with Amyloidosis, Familial Amyloidotic Polyneuropathy, Scrapie, Creutzfeldt-Jacob Disease, Gerstmann Straussler-Scheinker Syndrome, Bovine Spongiform Encephalitis, a Prion-mediated disease, and Huntington's Disease.

According to a specific embodiment, the neurodegenerative disease is Alzheimer's disease.

According to a specific embodiment, the neurodegenerative disease is Parkinson's disease.

The peptides of the present invention may be provided *per se* or as part of a pharmaceutical composition, where it is mixed with suitable carriers or excipients.

As used herein a "pharmaceutical composition" refers to a preparation of one or more of the active ingredients described herein with other chemical components such as physiologically suitable carriers and excipients. The purpose of a pharmaceutical composition is to facilitate administration of a compound to an organism.

Herein the term "active ingredient" refers to the peptides accountable for the biological effect.

Hereinafter, the phrases "physiologically acceptable carrier" and "pharmaceutically acceptable carrier" which may be interchangeably used refer to a carrier or a diluent that does not cause significant irritation to an organism and does not abrogate the biological activity and properties of the administered compound. An adjuvant is included under these phrases.

Herein the term "excipient" refers to an inert substance added to a pharmaceutical composition to further facilitate administration of an active ingredient. Examples, without limitation, of excipients include calcium carbonate, calcium phosphate, various sugars and types of starch, cellulose derivatives, gelatin, vegetable oils and polyethylene glycols.

Techniques for formulation and administration of drugs may be found in "Remington's Pharmaceutical Sciences," Mack Publishing Co., Easton, PA, latest edition.

Suitable routes of administration may, for example, include oral, rectal, transmucosal, especially transnasal, intestinal or parenteral delivery, including intramuscular, subcutaneous and intramedullary injections as well as intrathecal, direct intraventricular, intracardiac, e.g., into the right or left ventricular cavity, into the common coronary artery, intravenous, inrtaperitoneal, intranasal, or intraocular injections.

As described in length in the Examples section which follows, the peptides of the invention were able to protect against Alzheimer's and Parkinson's disease when administered directly into the brain such as by intrahippocampal (IH) intracerebroventricular injection (ICV), intracranial (IC) or intrathecal administration, essentially providing for a local mode of administration, each of which is contemplated herein.

Conventional approaches for drug delivery to the central nervous system (CNS) include: neurosurgical strategies (e.g., intracerebral injection or intracerebroventricular infusion); molecular manipulation of the agent (e.g., production of a chimeric fusion protein that comprises a transport peptide that has an affinity for an endothelial cell surface molecule in combination with an agent that is itself incapable of crossing the BBB) in an attempt to exploit one of the endogenous transport pathways of the BBB; pharmacological strategies designed to increase the lipid solubility of an agent (e.g., conjugation of water-soluble agents to lipid or cholesterol carriers); and the transitory disruption of the integrity of the BBB by hyperosmotic disruption (resulting from the infusion of a mannitol solution into the carotid artery or the use of a biologically active agent such as an angiotensin peptide). However, each of these strategies has limitations, such as the inherent risks associated with an invasive surgical procedure, a size limitation imposed by a limitation inherent in the endogenous transport systems, potentially undesirable biological side effects associated with the systemic administration of a chimeric molecule comprised of a carrier motif that could be active outside of the CNS, and the possible risk of brain damage within regions of the brain where the BBB is disrupted, which renders it a suboptimal delivery method.

Pharmaceutical compositions of the present invention may be manufactured by processes well known in the art, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries, which facilitate processing of the active ingredients into preparations which, can be used pharmaceutically. Proper formulation is dependent upon the route of administration chosen.

For injection, the active ingredients of the pharmaceutical composition may be formulated in aqueous solutions, preferably in physiologically compatible buffers such as Hank's solution, Ringer's solution, or physiological salt buffer. For transmucosal administration, penetrants appropriate to the barrier to be permeated are used in the formulation. Such penetrants are generally known in the art.

For oral administration, the pharmaceutical composition can be formulated readily by combining the active compounds with pharmaceutically acceptable carriers well known in the art. Such carriers enable the pharmaceutical composition to be formulated as tablets, pills, dragees, capsules, liquids, gels, syrups, slurries, suspensions, and the like, for oral ingestion by a patient. Pharmacological preparations for oral use can be made using a solid excipient, optionally grinding the resulting mixture, and processing the mixture of granules, after adding suitable auxiliaries if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carbomethylcellulose; and/or physiologically acceptable polymers such as polyvinylpyrrolidone (PVP). If desired, disintegrating agents may be added, such as cross-linked polyvinyl pyrrolidone, agar, or alginic acid or a salt thereof such as sodium alginate.

Dragee cores are provided with suitable coatings. For this purpose, concentrated sugar solutions may be used which may optionally contain gum arabic, talc, polyvinyl pyrrolidone, carbopol gel, polyethylene glycol, titanium dioxide, lacquer solutions and suitable organic solvents or solvent mixtures. Dyestuffs or pigments may be added to the tablets or dragee coatings for identification or to characterize different combinations of active compound doses.

Pharmaceutical compositions which can be used orally, include push-fit capsules made of gelatin as well as soft, sealed capsules made of gelatin and a plasticizer, such as glycerol or sorbitol. The push-fit capsules may contain the active ingredients in admixture with filler such as lactose, binders such as starches, lubricants such as talc or magnesium stearate and, optionally, stabilizers. In soft capsules, the active ingredients may be dissolved or suspended in suitable liquids, such as fatty oils, liquid paraffin, or liquid polyethylene glycols. In addition, stabilizers may be added. All formulations for oral administration should be in dosages suitable for the chosen route of administration.

For buccal administration, the compositions may take the form of tablets or lozenges formulated in conventional manner.

For administration by nasal inhalation, the active ingredients for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit may be determined by providing a valve to deliver a metered amount. Capsules and cartridges of, e.g., gelatin for use in a dispenser may be formulated containing a powder mix of the compound and a suitable powder base such as lactose or starch.

The pharmaceutical composition described herein may be formulated for parenteral administration, e.g., by bolus injection or continuous infusion. Formulations for injection may be presented in unit dosage form, e.g., in ampoules or in multidose containers with optionally, an added preservative. The compositions may be suspensions, solutions or emulsions in oily or aqueous vehicles, and may contain formulatory agents such as suspending, stabilizing and/or dispersing agents.

Pharmaceutical compositions for parenteral administration include aqueous solutions of the active preparation in water-soluble form. Additionally, suspensions of the active ingredients may be prepared as appropriate oily or water based injection suspensions. Suitable lipophilic solvents or vehicles include fatty oils such as sesame oil, or synthetic fatty acids esters such as ethyl oleate, triglycerides or liposomes. Aqueous injection suspensions may contain substances, which increase the viscosity of the suspension, such as sodium carboxymethyl cellulose, sorbitol or dextran. Optionally, the suspension may also contain suitable stabilizers or agents which increase the solubility of the active ingredients to allow for the preparation of highly concentrated solutions.

Alternatively, the active ingredient may be in powder form for constitution with a suitable vehicle, e.g., sterile, pyrogen-free water based solution, before use.

The pharmaceutical composition of the present invention may also be formulated in rectal compositions such as suppositories or retention enemas, using, e.g., conventional suppository bases such as cocoa butter or other glycerides.

Pharmaceutical compositions suitable for use in context of the present invention include compositions wherein the active ingredients are contained in an amount effective to achieve the intended purpose. More specifically, a therapeutically effective amount means an amount of active ingredients (CD44 peptides) effective to prevent, alleviate or ameliorate symptoms of a disorder (e.g., Parkinson's Disease, Alzheimer's disease) or prolong the survival of the subject being treated.

Determination of a therapeutically effective amount is well within the capability of those skilled in the art, especially in light of the detailed disclosure provided herein.

For any preparation, the therapeutically effective amount or dose can be estimated initially from in vitro and cell culture assays. For example, a dose can be formulated in animal models to achieve a desired concentration or titer. Such information can be used to more accurately determine useful doses in humans.

Toxicity and therapeutic efficacy of the active ingredients described herein can be determined by standard pharmaceutical procedures in vitro, in cell cultures or experimental animals. The data obtained from these in vitro and cell culture assays and animal studies can be used in formulating a range of dosage for use in human. The dosage may vary depending upon the dosage form employed and the route of administration utilized. The exact formulation, route of administration and dosage can be chosen by the individual physician in view of the patient's condition. (See e.g., Fingl, et al., 1975, in "The Pharmacological Basis of Therapeutics", Ch. 1 p.1).

Dosage amount and interval may be adjusted individually to brain or blood levels of the active ingredient are sufficient to induce or suppress the biological effect (minimal effective concentration, MEC). The MEC will vary for each preparation, but can be estimated from in vitro data. Dosages necessary to achieve the MEC will depend on individual characteristics and route of administration. Detection assays can be used to determine plasma concentrations.

Depending on the severity and responsiveness of the condition to be treated, dosing can be of a single or a plurality of administrations, with course of treatment lasting from several days to several weeks or until cure is effected or diminution of the disease state is achieved.

The amount of a composition to be administered will, of course, be dependent on the subject being treated, the severity of the affliction, the manner of administration, the judgment of the prescribing physician, etc.

Compositions of the present invention may, if desired, be presented in a pack or dispenser device, such as an FDA approved kit, which may contain one or more unit dosage forms containing the active ingredient. The pack may, for example, comprise metal or plastic foil, such as a blister pack. The pack or dispenser device may be accompanied by instructions for administration. The pack or dispenser may also be accommodated by a notice associated with the container in a form prescribed by a governmental agency regulating the manufacture, use or sale of pharmaceuticals, which notice is reflective of approval by the agency of the form of the compositions or human or veterinary administration. Such notice, for example, may be of labeling approved by the U.S. Food and Drug Administration for prescription drugs or of an approved product insert. Compositions comprising a preparation of the invention formulated in a compatible pharmaceutical carrier may also be prepared, placed in an appropriate container, and labeled for treatment of an indicated condition, as is further detailed above.

As used herein the term "about" refers to ± 10 %.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

### EXAMPLES

Reference is now made to the following examples, which together with the above descriptions illustrate some embodiments of the invention in a non limiting fashion.

Generally, the nomenclature used herein and the laboratory procedures utilized in the present invention include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Culture of Animal Cells - A Manual of Basic Technique" by Freshney, Wiley-Liss, N. Y. (1994), Third Edition; "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1996). Other general references are provided throughout this document. The procedures therein are believed to be well known in the art and are provided for the convenience of the reader.

### EXAMPLE 1

### Identification of active Peptides from exons 6 and 10 of CD44

### Materials and Methods

All peptides were synthesized by LifeTein (South Pleinfield, NJ, USA) at >95% purity. N2A mouse neuroblastoma and SK-N-SH human neuroblastoma (ATCC) were maintained in Dulbecco's modified Eagle's medium supplemented with 10 % fetal calf serum, L-Glut and 1 % Penicillin-Streptomycin (Beit Haemek, Israel). Cells were maintained in an incubator at 37 °C with 5 % CO₂. SK-N-SH cells were treated with 3 µM RA (Sigma-Aldrich) for 5 days prior to each experiment to allow the cells to differentiate towards neuronal cells. Cells were grown in 24 wells plate and treated with Aβ peptides for 48 hrs or MPTP (Sigma-Aldrich, 24 hrs) afterwhich they were subjected to to the XTT viability assay. The XTT viability assay is based on the ability of metabolic active cells to reduce the tetrazolium salt XTT to orange colored compounds of formazan. The intensity of the water soluble dye is proportional to the number of metabolic active cells. XTT (Beit Haemek, Israel) was added to the cells following treatment after 1:3 dilution with growth medium and incubated for 1-2 hours. Absorbance was measured at 420 nm. The viability assay was followed by caspase 3 assay (EnzChek Caspase3 Assay kit, Invitrogen, Carlsbad, Ca, USA) according to the manufacture's instructions.

### Results

In vitro and in vivo loss of function experiments using siRNA, indicate that CD44V6 and CD44V10 are playing a role in neurodegenerative disorders such as AD, PD and ALS (unpublished data). CD44S and splice variant isoforms were shown to participate in multiple protein-protein interactions, including in signal transduction pathways (reviewed by Ponta et al [9]). Therefore the present inventors envisioned that such interactions may mediate CD44V6 and CD44V10 function in neuronal cell death and that peptides derived from V6 or V10 exons sequences may serve as an agent for disruption of these interactions. Indeed it was shown that a small pentapeptide (NRWHE- SEQ ID NO: 1) derived from the human V6 sequence is capable of inhibiting hepatocyte growth factor (HGF) signal transduction through Met tyrosine kinase receptor [24].

In order to identify conserved sequences that may help to identify such peptides, the present inventors have made a multiple species sequence alignment for both V6 and V10 sequences (Figure 1). Based on the conserved sequences, synthesized several peptides that cover different regions in V6 and V10 exons were synthesized (Figure 2). The peptides were tested for their effect on cell death of neuroblastoma cell lines N2A (mouse) and SK-N-SH (human). As an example shown in Figure 3, the effect of two peptides derived from mouse V6 sequence and two peptides derived from the human V10 sequence was tested, at three concentrations, on cell death of SK-N-SH cells induced by Aβ 1-42 peptide. Cellular viability was measured by reduction of XTT. Shown are the relative viability percentage as 100% defined as the viability of the none-treated cells. All 4 peptides were found to confer at least partial protection to the cells compared to no peptide control (Figure 3). Surprisingly, the protection conferred the V6 peptides was maximal at a concentration as low as 20 pM and the degree of protection was reduced at higher concentrations. Additional peptides were further synthesized (6-21 amino acids, Figure 2) derived from mouse V6 and human V10 sequences and their protection activity from Aβ 25-35 toxicity in N2A cells was compared (Figures 4A-B). Aβ 25-35 mimics the toxicological and aggregational properties of the full-length peptide with increased potency [25]. At 1 nM concentration, most of the peptides protected the cells, at least partially, as measured by both viability assay (XTT, Figure 4A) and caspase 3 activity (as marker for apoptosis induction, Figure 4B). These peptides were also tested for protection from cell death induced by 1-methyl-4-phenyl-1,2,3,6-tetrahydropyridine (MPTP), a PD modeling neurotoxin which is metabolized to the toxic cation 1-methyl-4-phenylpyridinium (MPP⁺). Indeed some of the peptides were found also to have a protective effect against MPTP at 1 µM compared to control cells (without peptide). Results are presentedin Figures 5A-B.

### EXAMPLE 2

### Structural/functional analyses of CD44 peptides

### Materials and methods

All methods are the same as in Example 1 above. Peptides and 6-hydroxydopamine were purchased from Sigma-Aldrich (St. Lewis, USA).

### Results

These finding prompted the present inventors to expand the peptide screen to smaller peptides derived from conserved regions in V6B, V10A and V10B human sequences (Table 1).

**Table 1 (for Figures 6 and 7)**

| **Name** | **Sequence** | **SEQ ID NO:** |
|---|---|---|
| V6B1 | TPKEDSH | 16 |
| V6B1_C4 | EDSH | 17 |
| V10A/B_L | PVTSAKTGSFGVTAVTV | 18 |
| V10A/B_S | PVTSAKTGSFG | 19 |
| V10A2_C7 | PVTSAKT | 20 |
| V10A2_C10 | TFIPVTSAKT | 21 |
| V10A1_N12 | TTLLEGYTSHYP | 22 |
| V10A1_N8 | TTLLEGYT | 23 |
| V10A1_N6 | TTLLEG | 24 |
| V10A1_N+2_6 | LLEGYT | 25 |
| V10A1_N+4 (also referred to as P26) | EGYTSHYP | 26 |
| V10A1_EGYT | EGYT | 27 |
| V10B3_N7 | SLSGDQDT | 28 |
| V10B3_N6 | SLSGDQD | 29 |
| V10B3_N5 | SLSGD | 30 |
| V10B2.5_6 | NVNRSL | 31 |
| V10B2.5_9 | NVNRSLSGD | 32 |
| V10B2_N10 | DSNSNVNRSL | 33 |
| V10B1_8 (also referred to as P34) | FGVTAVTV | 34 |
| V10B1_7 | FGVTAVT | 35 |

These peptides were screened for their protective effect on 6-hydroxydopamine (6-OHDA) treated N2A cells at 1 pM concentration. 6-OHDA is a dopamine analogue that is commonly used in model systems to mimic Parkinson's disease in vitro and in vivo. 6-OHDA induces apoptosis through release of reactive oxygen species (ROS) and by a possible direct effect on the mitochondrial respiratory chain. The results show differential effect of the peptides on cellular viability and caspase 3 activation on 6-OHDA treated cells (Figures 6A-B). The protective effect of some of the peptides was tested in a wide dose range. For example, for the 8 mer peptide V10A1_N+4, the optimal protective dose was found to be around the fM concentration (Figure 6C). The peptide panel was also tested for their effect on Aβ (25-35) induced toxicity in SK-N-SH human neuroblastoma cells at pM concentration. The profile of peptides effective against the Aβ stress was strikingly different than the 6-OHDA-effective peptides (Figures 7A-B).

### EXAMPLE 3

### In vivo effect of some peptides of the invention in an in vivo Parkinson's model

### Materials and Methods

C57BL mice (all male, age 8-12 weeks, obtained from Harlan Laboratories Israel) were administered with 18 mg/kg MPTP (Sigma-Aldrich) at a dosage volume of 100µl/mouse by intraperitoneal (IP) injection twice daily, 3 hrs apart on days 1 and 2. On study day 0 and for 8 consecutive days, mice were administered intranasally with PBS (vehicle) or one of the peptides at 1 mg/kg in 12 µl/mouse. All peptides used in in vivo studies were synthesized by LifeTein and were modified with N terminal acetylation and C terminal amidation. For intranasal instillation, each mouse was mildly anesthetized (2.5% Isoflurane) then restrained and held with the neck parallel to the table while a total volume of 12µl was administered into the nostril. Six (6) µl was administered to the left nostril as two 3µl drops, followed by a 15 sec hold, and 6 µl was administered to the right nostril as two 3 µl drops, followed by a 15 seconds hold. On day 8 the animals were euthanized by cervical dislocation. I mmediately after euthanasia, the brains were removed and the striata dissected (left and right striatum were pooled), weighed and frozen in dry ice. The striata samples were homogenized in a solution containing 0.1 M perchloric acid and 10 ng/ml 3,4 dihydroxybenzylamine (DHBA) by 5 seconds sonication at 80 W. The supernatants of each tissue extract were injected directly to HPLC pump (Jasco PU- 2080Plus) onto a reverse phase column (GL-Science, Inertsil ODS-2 5um 4.6x150mm at room temperature) coupled to an electrochemical detector Coulochem II ESA with a conditioning cell model 5021 and analytical cell model 5011.The working potential was set to 0.35V on the conditioning cell and 0.1V and - 0.35V on the analytical cell. The mobile phase was 0.05M monobasic sodium phosphate ,with 80mg/L EDTA, 125 mg/L heptane sulfonic acid, 55ml of methanol and 50ml of acetonitrile pH=2.7. The flow rate was 1.5 ml/min. The dopamine, DOPAC and HVA values were normalized to the lysate protein concentration (BCA kit, Pierce).

### Results

The most effective peptides against MPTP and 6-OHDA were selected for in vivo PD model namely MPTP injection in mice. In this model, repeated itraperitoneal injections of MPTP (18 mg/kg at 3-h intervals on two consecutive days) result in dopaminergic neuronal death in the substantia nigra and dopamine depletion of the striatum. The peptides (or vehicle) were applied twice daily by intranasal instillation at 1 mg/kg starting 1 day prior to exposure to MPTP and continued until the end of the study. 7 days after the injection of MPTP mice were sacrificed and striatal levels of dopamine (DA), 3,2-dihydroxyphenylacetic acid (DOPAC) and homovanillic acid (HVA) were evaluated by HPLC. 9 peptides were tested in 8 groups and the results of DA, DOPAC and HVA levels are shown in Table 2.

Specifically Table 2 illustrates the effect of human V6 and V10-derived peptides (1 mg/kg, intranasal administration) on the striatal level of dopamine and metabolites in MPTP-treated mice. Mice were treated according to the protocol described in the Materials and Methods. Striatal levels of DA, DOPAC and HVA were determined by HPLC, divided by the total protein content value and normalized to the levels obtained in vehicle treated mice. Groups 1 and 3 treated with 1:1 mix of normal designated and retro-inverso analog (D amino acids at reversed sequence). Group 6 was treated with 1:1 mix of 10B2.5_6 and 10B2.5_9 peptides.

**Table 2**

| | ***SEQ ID NO*** | ***Name of peptide*** | ***DA*** | ***DOPAC*** | ***HVA*** |
|---|---|---|---|---|---|
| 1 | 17 | hV6B1_C4 | 20.7 | 41.7 | 16.6 |
| 2 | 19 | V10A/B_S | 26.6 | 27.2 | 13.7 |
| 3 | 24 | V10A1_N6 | 17.7 | 24.3 | -0.7 |
| 4 | 26 | V10A1_N+4 | 58.0* | 61.8* | 28.5 |
| 5 | 15 | hV10B3 | 18.1 | 22.5 | 44.2* |
| 6 | 31 and 32 | 10B2.5_6+10B2.5_9 | 13.6 | 35.1 | 53.0* |
| 7 | 34 | 10B1_8 | 46.6* | 75.9** | 83.3** |
| 8 | 12 | HV10A2 | 24.7 | 54.8* | 73.4** |

| | | | | | |
|---|---|---|---|---|---|
| *p value <0.05 **p value <0.01 | | | | | |

Treatment with two 8-mer, V10-derived peptides namely V10A1_N+4 (SEQ ID NO: 26) and 10B1_8 (SEQ ID NO: 34) had a significantly positive effect on striatal DA level compared to the vehicle control. These peptides, as well as three other peptides (hV10B3 - SEQ ID NO: 15; V10A2 - SEQ ID NO:12; 10B2.5_6 - SEQ ID NO: 31; and 10B2.5_9 - SEQ ID NO: 32), also increased significantly the levels of at least one of the 2 DA metabolites, DOPAC and HVA (Table 2, above). These results indicate that peptides derived from CD44 V10 are able to protect dopaminergic neurons from MPTP in vivo and suggest that these peptides be developed as novel drugs for Parkinson's disease.

### EXAMPLE 4

### Identification of active subsequences within the Peptides of some embodiments of the invention

### Results

V10A1_N+4 (hereiunder "P26", SEQ ID NO: 26) was chosen for further structure-function analysis. Peptides which were derived from this peptide were synthesized and shown on Table 3, below.

**Table 3**

| Name | SEQ ID NO: | Sequence |
|---|---|---|
| 26-1 | 36 | EGYTSHY |
| 26-2 | 37 | EGYTSH |
| 26-3 | 38 | EGYTS |
| 26-4 | 39 | GYTSHYP |
| 26-5 | 40 | YTSHYP |
| 26-6 | 41 | TSHYP |
| 26-7 | 42 | GYTSHY |
| 26-8 | 43 | QGYTSHYP |
| 26-9 | 44 | EGYTSAYP |
| 26-RI | 45 | *P*Y*H*S*T*Y*G*E |
| 26-R | 46 | PYHSTYGE |

| | | |
|---|---|---|
| * D amino acid | | |

The peptides shown on Table 3 were tested for their effect in protecting N2A from 6-OHDA (Figure 8). This data indicates that some modifications in the P26 sequence can be tolerated while maintaining at least partial activity. Such modifications include C-terminus truncation (26-1, 26-2 and 26-3), replacement of the N-terminal glutamic acid residue with glutamine and histidine at position 7 with alanine (26-8 and 26-9, respectively).

Most interestingly, a retro-inverso (RI, in which the primary sequence is reversed and D- rather than L- amino acids are used) P26 derivative (26-RI, SEQ ID NO: 45) also retain its neuroprotection activity. It is postulated that a retro-inverso peptides assume a side chain topology, in its extended conformation, similar to that of its native L-sequence and retaining the biological activities of the parent molecule while fully resistant to proteolytic degradation [Chorev, M. and M. Goodman, Recent developments in retro peptides and proteins--an ongoing topochemical exploration. Trends Biotechnol, 1995. 13(10): p. 438-45]. Therefore, it is contemplated that a minimal sequence for mediating neuroprotection is set forth in SEQ ID NO: 47 STYG-X (where X is E or Q) or retro configuration of same, whereby every amino acid can be D or L.

### EXAMPLE 5

### intrahippocampal (IH)/intracerebroventricular (ICV) injection of V10A1 N+4 (P26) and P34 peptides protects rats from Aβ₍₁₋₄₂₎ damage

### Materials and Methods

**Animals -** Aadult male Sprague Dawley rats were obtained from the Laboratory Animal Center of University of South China, Hengyang, Hunan, China. After arrival, the rats were housed individually in a temperature- and humidity-controlled environment with *ad libitum* access to food and water. Animals were maintained on a 12 hr light/dark schedule, with lights on at 7 A.M. After being housed, the rats were handled (5-6 min per rat per day) for 1 week to habituate them to the experimenter. Experiments were conducted according to the *National Institutes of Health Guide for the Care and Use of Laboratory Animals*, and experimental protocols were approved by the University Animal Care and Use Committee.

**Drugs** - Aβ₍₁₋₄₂₎ was purchased from Sigma-Aldrich (USA). Peptides obtained from LifeTein (USA).

**Establishment of the Alzheimer disease (AD) model** - Aβ₍₁₋₄₂₎ (Sigma-Aldrich, USA), was dissolved in filtered PBS at the concentration of 6 µg/µl, and the solution kept at 37°C for 2 days before use. One microliter of the solution was injected by means of a microsyringe into the right hippocampus under sodium pentobarbital (55 mg/kg i.p.) anesthesia at the stereotaxic coordinates: AP=-3.6, ML=2.0, from Bregma and DV=3.0 from the skull. Control rats were injected with 1µl of PBS. In the IH/ICV study, 1µl of the peptide or PBS was also injected into the right hippocampus at the same stereotaxic coordinates.

**Peptide administration** - For ICV injection, a microinjection cannula was planted into the lateral cerebral ventricle according to the following stereotaxic coordinates: AP=-1, ML=1.6, DV=3.8. Twenty-four hours after Aβ injection, rats received the first ICV injection of PBS or peptides at the designated doses. In the subcutaneous (SC) study, daily SC injections of PBS or peptide solution in PBS was done at 1 ml/rat to a dose of 1 mg/kg and lasted for 21 days.

**Novel object recognition task -** The NOR task was tested 21 days after Aβ injection.

The training apparatus was a black Plexiglas box (50×50×40 cm) placed in a sound-attenuating cabinet which was located in a brightly lit and isolated room. Illumination was provided by a 15 W white house light mounted on the ceiling of cabinet, and a 65 dB background noise was supplied by a ventilation fan in the cabinet. The floor of the box was covered with sawdust. The objects used in the task were made of water-repellant materials such as glass and plastic with differences in shape and color. The sizes of the objects were about 6×6×8 cm. Two objects were always located in the back corners of the box. The location and objects were counterbalanced to control for any preferences that the rats might have had for one of the corners or of the objects. The behavioral procedure involved two phases: training and retention test. During the training trial, the rat was placed in the box and allowed to explore two identical objects for 10 min and the total time spent exploring both objects was recorded. Exploration of an object was defined as pointing the nose to the object at a distance of <1 cm and/or touching it with the nose. The sawdust was stirred and the box and the objects were cleaned with 40% ethanol solution between trials. Twenty-four hours after training trial (retention test trial), one copy of the familiar object and a new object were placed in the same location as stimuli during the training phase. The rat was placed in the box for 3 min and the time spent exploring each object and the total time spent exploring both objects were recorded. The discrimination index used to assess memory was calculated as the difference in time exploring the novel and familiar object, expressed as the ratio of the total time spent exploring both objects.

**Morris water maze test -** consisted of a circular water tank (200 cm diameter, 60 cm height) filled with water (25±1 °C) to a depth of 40 cm. Four equally spaced locations around the edge of the pool were used as start points, which divided the pool into 4 quadrants. An escape platform (10 cm in diameter) was placed in the pool 2 cm below the surface of water. The escape platform was placed in the middle of one of the randomly selected quadrants of the pool and kept in the same position throughout the entire experiment. Before the training started, the rats were allowed to swim freely into the pool for 120 s without platform. Animals received a training session consisting of 4 trials per session (once from each starting point) for 4 days, each trial having a ceiling time of 120 s and a trial interval of approximately 30 s. After climbing onto the hidden platform, the animals remained there for 30 s before commencement of the next trial. If the rat failed to locate the hidden platform within the maximum time of 120 s, it was gently placed on the platform and allowed to remain there for the same interval of time. The time taken to locate the hidden platform (latency in seconds) was measured. Twenty four hours after the acquisition phase, a probe test was conducted by removing the platform. Rats were allowed to swim freely in the pool for 120 s and the time spent in target quadrant, which had previously contained the hidden platform, was recorded. The time spent in the target quadrant indicated the degree of memory consolidation which had taken place after learning. The Morris water maze test was started 24 days after Aβ injection.

**Statistical Analyses** - Statistical analyses were performed using one-way ANOVA. Post-hoc comparisons were performed with the Fisher LSD Test (SigmaStat 3.2). All data were represented as mean ± SEM. Significant level was set at *p*<0.05.

### Results

In order to test the efficacy P26 and V10B1_8 (hereinunder P34) peptides in Alzheumer's disease (AD) model in vivo, these peptides were tested in Aβ₍₁₋₄₂₎ microinjection rat model [Soto, C., et al., Beta-sheet breaker peptides inhibit fibrillogenesis in a rat brain model of amyloidosis: implications for Alzheimer's therapy. Nat Med, 1998. 4(7): p. 822-6]. In this model, rats received a single microinjection of 6 µg Aβ₍₁₋₄₂₎ into the right hippocampus. At the same time, 1µl of peptide solution or PBS (vehicle) was also injected into the same location. This treatment was followed by daily ICV injection of the peptides at different doses or vehicle (PBS). 21 days after Aβ injection the rats were tested for the novel object recognition assay (NOR, Figure 9). The results show that P26 at 100 ng/rat and P34 at 10 and 100 ng/rat increased significantly the discrimination index (p<0.01). The data indicates that ICV/IH administration of the P26 and P34 peptides prevent the toxic effect of Aβ in vivo.

### EXAMPLE 6

### Peripheral injection of V10A1_N+4 (P26) peptide protects rats from Aβ₍₁₋₄₂₎ damage

### Material and Methods

Experimental procedures and subcutaneous injection (SC) is described in Example 5 above.

### Results

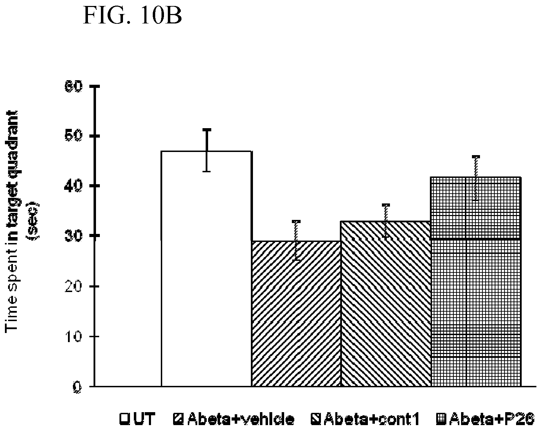

Parenteral administration of the peptides by subcutaneous (SC) injection was assayed for protecting the rats from Aβ toxicity. Therefore the Aβ₍₁₋₄₂₎ microinjection rat model was applied followed by daily SC injection of 1 mg/kg P26 peptide, as well as the control peptide (cont1: AVAVEAAG SEQ ID NO: 48, n=10-11). The 21 days injection period was followed by Morris water maze (MWM) and NOR memory assays. The results show that of the 3 SC-injected peptide only P26 improved significantly the behavior in both assays (Figures 10 and 11). These results demonstrate that the neuroprotection effect of the P26 peptide is seen also when the peptide is given by parenteral administration, suggesting that the peptide is able to cross the blood-brain barrier and reach sufficient concentration in the relevant brain regions.

### EXAMPLE 7

### pharmacokinetics of P26 and P26-IR

### Materials and Methods

The pharmacokinetics of the peptides was evaluated following SC peptide administration in male Sprague Dawley rats. Peptide solution was prepared using phosphate buffer saline (pH 7.4) as vehicle and was administered through subcutaneously at the dose of 1 mg/kg with dosing volume of 2 mL/kg.

Blood samples were collected at 0 (pre-dose) and 0.17, 0.5, 1, 2, 4, 8 and 24 hours following administration. At each time point, approximately 0.25 mL of blood was withdrawn through jugular vein of the cannulated rats and transferred to a pre-labeled microfuge tube containing 200 mM K₂EDTA (20 µL per mL of blood). Following sampling equal volume of heparinized saline was flushed in to catheter. Blood samples were centrifuged at 5000g for 5 minutes at 4 ± 2°C. All the plasma samples were stored below -70 °C until analysis. A fit-for-purpose LC-MS/MS method was used for the quantification of the peptides in plasma samples. The lower limit of quantification (LLOQ) was 22.34 ng/mL.

### Results

The pharmacokinetics of P26 and P26-IR were tested in rats by subcutaneous injection at 1 mg/kg (Figure 12). P26-RI demonstrated improved SC pharmacokinetics with an apparent Cₘₐₓ of 1227 ng/ml compared to 130 ng/ml for P12. This 10 fold improvement by P26-IR analogue, taken together with its activity in vitro, suggests that P26-IR can be used at lower doses compared to P26.

### REFERENCES

### (other references are recited in the application)

1. Querfurth, H.W. and F.M. LaFerla, Alzheimer's disease. N Engl J Med, 2010. 362(4): p. 329-44.
2. Glass, C.K., et al., Mechanisms underlying inflammation in neurodegeneration. Cell, 2010.140(6): p. 918-34.
3. Davie, C.A., A review of Parkinson's disease. Br Med Bull, 2008. 86: p. 109-27.
4. Naor, D., R.V. Sionov, and D. Ish-Shalom, CD44: structure, function, and association with the malignant process. Adv Cancer Res, 1997. 71: p. 241-319.
5. Ilangumaran, S., B. Borisch, and D.C. Hoessli, Signal transduction via CD44: role of plasma membrane microdomains. Leuk Lymphoma, 1999. 35(5-6): p. 455-69.
6. DeGrendele, H.C., et al., CD44 and its ligand hyaluronate mediate rolling under physiologic flow: a novel lymphocyte-endothelial cell primary adhesion pathway. J Exp Med, 1996.183(3): p. 1119-30.
7. DeGrendele, H.C., P. Estess, and M.H. Siegelman, Requirement for CD44 in activated T cell extravasation into an inflammatory site. Science, 1997. 278(5338): p. 672-5.
8. Ponta, H., D. Wainwright, and P. Herrlich, The CD44 protein family. Int J Biochem Cell Biol, 1998. 30(3): p. 299-305.
9. Ponta, H., L. Sherman, and P.A. Herrlich, CD44: from adhesion molecules to signalling regulators. Nat Rev Mol Cell Biol, 2003. 4(1): p. 33-45.
10. Golan, I., et al., Expression of extra trinucleotide in CD44 variant of rheumatoid arthritis patients allows generation of disease-specific monoclonal antibody. J Autoimmun, 2007. 28(2-3): p. 99-113.
11. Garin, T., et al., CD44 variant DNA vaccination with virtual lymph node ameliorates experimental autoimmune encephalomyelitis through the induction of apoptosis. J Neurol Sci, 2007. 258(1-2): p. 17-26.
12. Orian-Rousseau, V., et al., CD44 is required for two consecutive steps in HGF/c-Met signaling. Genes Dev, 2002. 16(23): p. 3074-86.
13. Tremmel, M., et al., A CD44v6 peptide reveals a role of CD44 in VEGFR-2 signaling and angiogenesis. Blood, 2009. 114(25): p. 5236-44.
14. Akiyama, H., et al., Morphological diversities of CD44 positive astrocytes in the cerebral cortex of normal subjects and patients with Alzheimer's disease. Brain Res, 1993. 632(1-2): p. 249-59.
15. Moretto, G., R.Y. Xu, and S.U. Kim, CD44 expression in human astrocytes and oligodendrocytes in culture. J Neuropathol Exp Neurol, 1993. 52(4): p. 419-23.
16. Bignami, A. and R. Asher, Some observations on the localization of hyaluronic acid in adult, newborn and embryonal rat brain. Int J Dev Neurosci, 1992. 10(1): p. 45-57.
17. Kaaijk, P., et al., Differential expression of CD44 splice variants in the normal human central nervous system. J Neuroimmunol, 1997. 73(1-2): p. 70-6.
18. Asher, R. and A. Bignami, Hyaluronate binding and CD44 expression in human glioblastoma cells and astrocytes. Exp Cell Res, 1992. 203(1): p. 80-90.
19. Kang, W.S., et al., Differential regulation of osteopontin receptors, CD44 and the alpha(v) and beta(3) integrin subunits, in the rat hippocampus following transient forebrain ischemia. Brain Res, 2008. 1228: p. 208-16.
20. Ries, A., J.L. Goldberg, and B. Grimpe, A novel biological function for CD44 in axon growth of retinal ganglion cells identified by a bioinformatics approach. J Neurochem, 2007. 103(4): p. 1491-505.
21. Wang, X., et al., CD44 deficiency in mice protects brain from cerebral ischemia injury. J Neurochem, 2002. 83(5): p. 1172-9.
22. Lammich, S., et al., Presenilin-dependent intramembrane proteolysis of CD44 leads to the liberation of its intracellular domain and the secretion of an Abeta-like peptide. J Biol Chem, 2002. 277(47): p. 44754-9.
23. Pinner, E., M. Laudon, and N. Zisapel, *CD44 splice variants in neurodegenerative diseases.* WO/2009/007934, 2009.
24. Matzke, A., et al., A five-amino-acid peptide blocks Met- and Ron-dependent cell migration. Cancer Res, 2005. 65(14): p. 6105-10.
25. Varadarajan, S., et al., Different mechanisms of oxidative stress and neurotoxicity for Alzheimer's A beta(1--42) and A beta(25--35). J Am Chem Soc, 2001. 123(24): p. 5625-31.

## Claims

1. An isolated peptide consisting of a CD44V10 amino acid sequence selected from the group consisting of SEQ ID NOs: 12, 15, 26, 31, 32, 34 and 45.

2. A pharmaceutical composition comprising as an active agent an isolated peptide consisting of the isolated peptide of claim 1 and a pharmaceutically effective carrier.

3. The isolated peptide of claim 1 or pharmaceutical composition of claim 2 for use in treating a neurodegenerative disorder selected from the group consisting of Parkinson's disease, Multiple Sclerosis, ALS, multi-system atrophy, Alzheimer's disease, stroke, traumatic brain injury, progressive supranuclear palsy, fronto-temporal dementia with parkinsonism linked to chromosome 17 and Pick's disease.

4. The isolated peptide or pharmaceutical composition for use according to claim 3, wherein said isolated peptide consists of a CD44V10 amino acid sequence selected from the group consisting of SEQ ID NO: 26, 32 and 45.

5. The isolated peptide or pharmaceutical composition for use according to claim 3 or 4, wherein said neurodegenerative disorder is Parkinson's disease.

6. The isolated peptide or pharmaceutical composition for use according to claim 3 or 4, wherein said neurodegenerative disorder is Alzheimer's disease.

7. The isolated peptide, the isolated peptide for use or pharmaceutical composition of any of claims 1-6, wherein the peptide is attached to a cell penetrating agent.

8. The isolated peptide or pharmaceutical composition for use of any one of claims 3-7 by subcutaneous or intranasal delivery.

## Patentansprüche

1. Isoliertes Peptid bestehend aus einer CD44V10 Aminosäuresequenz, ausgewählt aus der Gruppe, bestehend aus SEQ ID NR.: 12, 15, 26, 31, 32, 34 und 45.

2. Pharmazeutische Zusammensetzung, umfassend als ein aktives Agens ein isoliertes Peptid bestehend aus dem isolierten Peptid nach Anspruch 1 und einem pharmazeutisch wirksamen Träger.

3. Isoliertes Peptid nach Anspruch 1 oder pharmazeutische Zusammensetzung nach Anspruch 2 zur Verwendung beim Behandeln einer neurodegenerativen Störung, ausgewählt aus der Gruppe bestehend aus Parkinson-Krankheit, Multipler Sklerose, ALS, Multisystematrophie, Alzheimer-Krankheit, Schlaganfall, traumatischer Hirnverletzung, progressiver supranukleärer Lähmung, frontotemporaler Demenz mit Parkinsonismus, verbunden mit Chromosom 17, und Pick-Krankheit.

4. Isoliertes Peptid oder pharmazeutische Zusammensetzung zur Verwendung nach Anspruch 3, wobei das isolierte Peptid aus einer CD44V10 Aminosäuresequenz besteht, ausgewählt aus der Gruppe, bestehend aus SEQ ID NR.: 26, 32 und 45.

5. Isoliertes Peptid oder pharmazeutische Zusammensetzung zur Verwendung nach Anspruch 3 oder 4, wobei die neurodegenerative Störung Parkinson-Krankheit ist.

6. Isoliertes Peptid oder pharmazeutische Zusammensetzung zur Verwendung nach Anspruch 3 oder 4, wobei die neurodegenerative Störung Alzheimer-Krankheit ist.

7. Isoliertes Peptid, das isolierte Peptid zur Verwendung oder pharmazeutischen Zusammensetzung nach einem der Ansprüche 1-6, wobei das Peptid an einem Zellenpenetrationsagens befestigt ist.

8. Isoliertes Peptid oder pharmazeutische Zusammensetzung zur Verwendung nach einem der Ansprüche 3-7 durch subkutane oder intranasale Verabreichung.

## Revendications

1. Un peptide isolé consistant en une séquence d'acide aminé CD44V10 choisie dans le groupe consistant en SEQ ID No: 12 15, 26, 31, 32, 34 et 45.

2. Une composition pharmaceutique comprenant en tant qu'agent actif un peptide isolé consistant en le peptide isolé de la revendication 1 et en un support efficace du point de vue pharmaceutique.

3. Le peptide isolé de la revendication 1 ou la composition pharmaceutique de la revendication 2 pour une utilisation dans le traitement des troubles neurodégénératifs du groupe des troubles consistant en la maladie de Parkinson, la sclérose en plaque, la sclérose latérale amyotrophique, l'atrophie multi-systémique, la maladie d'Alzheimer, les accidents vasculaires cérébraux, la lésion cérébrale traumatique, la paralysie supra-nucléaire progressive, la dégénérescence fronto-temporale avec le parkinsonisme lié au chromosome 17 et la maladie de Pick.

4. Le peptide isolé ou la composition pharmaceutique pour une utilisation selon la revendication 3, lequel peptide isolé consiste en une séquence d'acide aminé CD44V10 choisie dans le groupe consistant en SEQ ID No: 26, 32 et 45.

5. Le peptide isolé ou la composition pharmaceutique pour une utilisation selon la revendication 3 ou 4, dans lequel ledit trouble neurodégénératif est la maladie de Parkinson.

6. Le peptide isolé ou la composition pharmaceutique pour une utilisation selon la revendication 3 ou 4, dans lequel ledit trouble neurodégénératif est la maladie d'Alzheimer.

7. Le peptide isolé, le peptide isolé pour une utilisation ou une composition selon une quelconque des revendications 1 à 6, dans lequel le peptide est lié à un agent de pénétration de cellules.

8. Le peptide isolé ou la composition pharmaceutique pour une utilisation selon la une quelconque des revendications 3 à 7 pour une administration sous-cutanée ou intranasale.


### SEQUENCE LISTING

<110> Neurim Pharmaceuticals (1991) Ltd. Pinner , Elhanan Zisapel, Nava
<120> NEUROPROTECTIVE PEPTIDES
<130> 53555
<150> US 61/466,966
   <151> 2011-03-24
<160> 56
<170> PatentIn version 3.5
<210> 1
   <211> 5
   <212> PRT
   <213> artificial sequence
<220>
   <223> A pentapeptide derived from the human V6
<400> 1
   Asn Arg Trp His Glu
   1               5
<210> 2
   <211> 39
   <212> PRT
   <213> artificial sequence
<220>
   <223> Mouse V6 peptide
<400> 2
   Pro Asn Ser Thr Ala Glu Ala Ala Ala Thr Gln Gln Glu Thr Trp Phe
   1               5                   10                  15
   Gln Asn Gly Trp Gln Gly Lys Asn Pro Pro Thr Pro Ser Glu Asp Ser
               20                  25                  30
   His Val Thr Glu Gly Thr Thr
           35
<210> 3
   <211> 20
   <212> PRT
   <213> artificial sequence
<220>
   <223> Mouse V6A peptide
<400> 3
   Pro Asn Ser Thr Ala Glu Ala Ala Ala Thr Gln Gln Glu Thr Trp Phe
   1               5                   10                  15
   Gln Asn Gly Trp
               20
<210> 4
   <211> 13
   <212> PRT
   <213> artificial sequence
<220>
   <223> Mouse V6A1 peptide
<400> 4
   Pro Asn Ser Thr Ala Glu Ala Ala Ala Thr Gln Gln Glu
   1               5                   10
<210> 5
   <211> 6
   <212> PRT
   <213> artificial sequence
<220>
   <223> Mouse V6A2 peptide
<400> 5
   Trp Phe Gln Asn Gly Trp
   1               5
<210> 6
   <211> 12
   <212> PRT
   <213> artificial sequence
<220>
   <223> Mouse V6B peptide
<400> 6
   Thr Pro Ser Glu Asp Ser His Val Thr Glu Gly Thr
   1               5                   10
<210> 7
   <211> 7
   <212> PRT
   <213> artificial sequence
<220>
   <223> Mouse V6B1 peptide
<400> 7
   Thr Pro Ser Glu Asp Ser His
   1               5
<210> 8
   <211> 61
   <212> PRT
   <213> artificial sequence
<220>
   <223> Human V10 peptide
<400> 8
   Thr Thr Leu Leu Glu Gly Tyr Thr Ser His Tyr Pro His Thr Lys Glu
   1               5                   10                  15
   Ser Arg Thr Phe Ile Pro Val Thr Ser Ala Lys Thr Gly Ser Phe Gly
               20                  25                  30
   Val Thr Ala Val Thr Val Gly Asp Ser Asn Ser Asn Val Asn Arg Ser
           35                  40                  45
   Leu Ser Gly Asp Gln Asp Thr Phe His Pro Ser Gly Gly
       50                  55                  60
<210> 9
   <211> 28
   <212> PRT
   <213> artificial sequence
<220>
   <223> Human V10A peptide
<400> 9
   Thr Thr Leu Leu Glu Gly Tyr Thr Ser His Tyr Pro His Thr Lys Glu
   1 5 10 15
   Ser Arg Thr Phe Ile Pro Val Thr Ser Ala Lys Thr
   20 25
<210> 10
   <211> 33
   <212> PRT
   <213> artificial sequence
<220>
   <223> Human V10B peptide
<400> 10
   Gly Ser Phe Gly Val Thr Ala Val Thr Val Gly Asp Ser Asn Ser Asn
   1 5 10 15
   Val Asn Arg Ser Leu Ser Gly Asp Gln Asp Thr Phe His Pro Ser Gly
   20 25 30
   Gly
<210> 11
   <211> 16
   <212> PRT
   <213> artificial sequence
<220>
   <223> Human V10A1 peptide
<400> 11
   Thr Thr Leu Leu Glu Gly Tyr Thr Ser His Tyr Pro His Thr Lys Glu
   1 5 10 15
<210> 12
   <211> 15
   <212> PRT
   <213> artificial sequence
<220>
   <223> Human V10A2 peptide
<400> 12
   Thr Lys Glu Ser Arg Thr Phe Ile Pro Val Thr Ser Ala Lys Thr
   1 5 10 15
<210> 13
   <211> 18
   <212> PRT
   <213> artificial sequence
<220>
   <223> Human V10B1 peptide
<400> 13
   Gly Ser Phe Gly Val Thr Ala Val Thr Val Gly Asp Ser Asn Ser Asn
   1 5 10 15
   Val Asn
<210> 14
   <211> 21
   <212> PRT
   <213> artificial sequence
<220>
   <223> Human V10B2 peptide
<400> 14
   Asp Ser Asn Ser Asn Val Asn Arg Ser Leu Ser Gly Asp Gln Asp Thr
   1 5 10 15
   Phe His Pro Ser Gly
   20
<210> 15
   <211> 13
   <212> PRT
   <213> artificial sequence
<220>
   <223> Human V10B3 peptide
<400> 15
   Ser Leu Ser Gly Asp Gln Asp Thr Phe His Pro Ser Gly
   1 5 10
<210> 16
   <211> 7
   <212> PRT
   <213> Artificial sequence
<220>
   <223> Human V6B1 peptide
<400> 16
   Thr Pro Lys Glu Asp Ser His
   1 5
<210> 17
   <211> 4
   <212> PRT
   <213> Artificial sequence
<220>
   <223> Human V6B1_C4 peptide
<400> 17
   Glu Asp Ser His
   1
<210> 18
   <211> 17
   <212> PRT
   <213> Artificial sequence
<220>
   <223> Human V10A/B_L peptide
<400> 18
   Pro Val Thr Ser Ala Lys Thr Gly Ser Phe Gly Val Thr Ala Val Thr
   1 5 10 15
   Val
<210> 19
   <211> 11
   <212> PRT
   <213> Artificial sequence
<220>
   <223> Human V10A/B_S peptide
<400> 19
   Pro Val Thr Ser Ala Lys Thr Gly Ser Phe Gly
   1 5 10
<210> 20
   <211> 7
   <212> PRT
   <213> Artificial sequence
<220>
   <223> Human V10A2_C7 peptide
<400> 20
   Pro Val Thr Ser Ala Lys Thr
   1 5
<210> 21
   <211> 10
   <212> PRT
   <213> Artificial sequence
<220>
   <223> Human V10A2_C10 peptide
<400> 21
   Thr Phe Ile Pro Val Thr Ser Ala Lys Thr
   1 5 10
<210> 22
   <211> 12
   <212> PRT
   <213> Artificial sequence
<220>
   <223> Human V10A1_N12 peptide
<400> 22
   Thr Thr Leu Leu Glu Gly Tyr Thr Ser His Tyr Pro
   1 5 10
<210> 23
   <211> 8
   <212> PRT
   <213> Artificial sequence
<220>
   <223> Human V10A1_N8 peptide
<400> 23
   Thr Thr Leu Leu Glu Gly Tyr Thr
   1 5
<210> 24
   <211> 6
   <212> PRT
   <213> Artificial sequence
<220>
   <223> Human V10A1_N6 peptide
<400> 24
   Thr Thr Leu Leu Glu Gly
   1 5
<210> 25
   <211> 6
   <212> PRT
   <213> Artificial sequence
<220>
   <223> Human V10A1_N+2_6 peptide
<400> 25
   Leu Leu Glu Gly Tyr Thr
   1 5
<210> 26
   <211> 8
   <212> PRT
   <213> Artificial sequence
<220>
   <223> Human V10A1_N+4 peptide (also referred to as P26)
<400> 26
   Glu Gly Tyr Thr Ser His Tyr Pro
   1 5
<210> 27
   <211> 4
   <212> PRT
   <213> Artificial sequence
<220>
   <223> Human V10A1_EGYT peptide
<400> 27
   Glu Gly Tyr Thr
   1
<210> 28
   <211> 8
   <212> PRT
   <213> Artificial sequence
<220>
   <223> Human V10B3_N7 peptide
<400> 28
   Ser Leu Ser Gly Asp Gln Asp Thr
   1 5
<210> 29
   <211> 7
   <212> PRT
   <213> Artificial sequence
<220>
   <223> Human V10B3_N6 peptide
<400> 29
   Ser Leu Ser Gly Asp Gln Asp
   1 5
<210> 30
   <211> 5
   <212> PRT
   <213> Artificial sequence
<220>
   <223> Human V10B3_N5 peptide
<400> 30
   Ser Leu Ser Gly Asp
   1 5
<210> 31
   <211> 6
   <212> PRT
   <213> Artificial sequence
<220>
   <223> Human V10B2.5_6 peptide
<400> 31
   Asn Val Asn Arg Ser Leu
   1 5
<210> 32
   <211> 9
   <212> PRT
   <213> Artificial sequence
<220>
   <223> Human V10B2.5_9 peptide
<400> 32
   Asn Val Asn Arg Ser Leu Ser Gly Asp
   1 5
<210> 33
   <211> 10
   <212> PRT
   <213> Artificial sequence
<220>
   <223> Human V10B2_N10 peptide
<400> 33
   Asp Ser Asn Ser Asn Val Asn Arg Ser Leu
   1 5 10
<210> 34
   <211> 8
   <212> PRT
   <213> Artificial sequence
<220>
   <223> Human V10B1_8 peptide (also referred to as P34)
<400> 34
   Phe Gly Val Thr Ala Val Thr Val
   1 5
<210> 35
   <211> 7
   <212> PRT
   <213> Artificial sequence
<220>
   <223> Human V10B1_7 peptide
<400> 35
   Phe Gly Val Thr Ala Val Thr
   1 5
<210> 36
   <211> 7
   <212> PRT
   <213> Artificial sequence
<220>
   <223> Human 26-1 peptide
<400> 36
   Glu Gly Tyr Thr Ser His Tyr
   1 5
<210> 37
   <211> 6
   <212> PRT
   <213> Artificial sequence
<220>
   <223> Human 26-2 peptide
<400> 37
   Glu Gly Tyr Thr Ser His
   1 5
<210> 38
   <211> 5
   <212> PRT
   <213> Artificial sequence
<220>
   <223> Human 26-3 peptide
<400> 38
   Glu Gly Tyr Thr Ser
   1 5
<210> 39
   <211> 7
   <212> PRT
   <213> Artificial sequence
<220>
   <223> Human 26-4 peptide
<400> 39
   Gly Tyr Thr Ser His Tyr Pro
   1 5
<210> 40
   <211> 6
   <212> PRT
   <213> Artificial sequence
<220>
   <223> Human 26-5 peptide
<400> 40
   Tyr Thr Ser His Tyr Pro
   1 5
<210> 41
   <211> 5
   <212> PRT
   <213> Artificial sequence
<220>
   <223> Human 26-6 peptide
<400> 41
   Thr Ser His Tyr Pro
   1 5
<210> 42
   <211> 6
   <212> PRT
   <213> Artificial sequence
<220>
   <223> Human 26-7 peptide
<400> 42
   Gly Tyr Thr Ser His Tyr
   1 5
<210> 43
   <211> 8
   <212> PRT
   <213> Artificial sequence
<220>
   <223> Human 26-8 peptide
<400> 43
   Gln Gly Tyr Thr Ser His Tyr Pro
   1 5
<210> 44
   <211> 8
   <212> PRT
   <213> Artificial sequence
<220>
   <223> Human 26-9 peptide
<400> 44
   Glu Gly Tyr Thr Ser Ala Tyr Pro
   1               5
<210> 45
   <211> 8
   <212> PRT
   <213> Artificial sequence
<220>
   <223> Human 26-RI peptide
<220>
   <221> misc_feature
   <222> (1)..(8)
   <223> D stereoisomer
<400> 45
   Pro Tyr His Ser Thr Tyr Gly Glu
   1               5
<210> 46
   <211> 8
   <212> PRT
   <213> Artificial sequence
<220>
   <223> Human 26-R peptide
<400> 46
   Pro Tyr His Ser Thr Tyr Gly Glu
   1               5
<210> 47
   <211> 5
   <212> PRT
   <213> artificial sequence
<220>
   <223> A minimal peptide sequence for mediating neuroprotection
<220>
   <221> misc_feature
   <222> (1)..(5)
   <223> Can be D or L stereoisomer
<220>
   <221> misc_feature
   <222> (5)..(5)
   <223> X can be E or Q
<400> 47
   Ser Thr Tyr Gly Xaa
   1 5
<210> 48
   <211> 8
   <212> PRT
   <213> artificial sequence
<220>
   <223> A control peptide
<400> 48
   Ala Val Ala Val Glu Ala Ala Gly
   1 5
<210> 49
   <211> 19
   <212> PRT
   <213> artificial sequence
<220>
   <223> An amino acid sequence missing from the CD44V10
<400> 49
<210> 50
   <211> 19
   <212> PRT
   <213> artificial sequence
<220>
   <223> An amino acid sequence missing from the CD44V10
<400> 50
   Asn Ser Asn Val Asn Arg Ser Leu Ser Gly Asp Gln Asp Thr Phe His
   1 5 10 15
   Pro Ser Gly
<210> 51
   <211> 19
   <212> PRT
   <213> artificial sequence
<220>
   <223> An amino acid sequence missing from the CD44 V6
<400> 51
   Asp Ser Thr Asp Arg Ile Pro Ala Thr Ile Gln Ala Thr Pro Ser Ser
   1               5                   10                  15
   Thr Thr Glu
<210> 52
   <211> 19
   <212> PRT
   <213> artificial sequence
<220>
   <223> An amino acid sequence missing from the CD44 V6
<400> 52
   Asp Ser His Ser Thr Thr Gly Thr Ala Gly Asp Gln Asp Thr Phe His
     1               5                   10                  15
   Pro Ser Gly
<210> 53
   <211> 742
   <212> PRT
   <213> Homo sapiens
<400> 53
   Met Asp Lys Phe Trp Trp His Ala Ala Trp Gly Leu Cys Leu Val Pro
   1               5                   10                  15
   Leu Ser Leu Ala Gln Ile Asp Leu Asn Ile Thr Cys Arg Phe Ala Gly
               20                  25                  30
   Val Phe His Val Glu Lys Asn Gly Arg Tyr Ser Ile Ser Arg Thr Glu
           35                  40                  45
   Ala Ala Asp Leu Cys Lys Ala Phe Asn Ser Thr Leu Pro Thr Met Ala
       50                  55                  60
   Gln Met Glu Lys Ala Leu Ser Ile Gly Phe Glu Thr Cys Arg Tyr Gly
   65                  70                  75                  80
   Phe Ile Glu Gly His Val Val Ile Pro Arg Ile His Pro Asn Ser Ile
                   85                  90                  95
   Cys Ala Ala Asn Asn Thr Gly Val Tyr Ile Leu Thr Ser Asn Thr Ser
               100                 105                 110
   Gln Tyr Asp Thr Tyr Cys Phe Asn Ala Ser Ala Pro Pro Glu Glu Asp
           115                 120                 125
   Cys Thr Ser Val Thr Asp Leu Pro Asn Ala Phe Asp Gly Pro Ile Thr
       130                 135                 140
   Ile Thr Ile Val Asn Arg Asp Gly Thr Arg Tyr Val Gln Lys Gly Glu
   145                 150                 155                 160
   Tyr Arg Thr Asn Pro Glu Asp Ile Tyr Pro Ser Asn Pro Thr Asp Asp
                   165                 170                 175
   Asp Val Ser Ser Gly Ser Ser Ser Glu Arg Ser Ser Thr Ser Gly Gly
               180                 185                 190
   Tyr Ile Phe Tyr Thr Phe Ser Thr Val His Pro Ile Pro Asp Glu Asp
   195 200 205
   Ser Pro Trp Ile Thr Asp Ser Thr Asp Arg Ile Pro Ala Thr Thr Leu
   210 215 220
   Met Ser Thr Ser Ala Thr Ala Thr Glu Thr Ala Thr Lys Arg Gln Glu
   225 230 235 240
   Thr Trp Asp Trp Phe Ser Trp Leu Phe Leu Pro Ser Glu Ser Lys Asn
   245 250 255
   His Leu His Thr Thr Thr Gln Met Ala Gly Thr Ser Ser Asn Thr Ile
   260 265 270
   Ser Ala Gly Trp Glu Pro Asn Glu Glu Asn Glu Asp Glu Arg Asp Arg
   275 280 285
   His Leu Ser Phe Ser Gly Ser Gly Ile Asp Asp Asp Glu Asp Phe Ile
   290 295 300
   Ser Ser Thr Ile Ser Thr Thr Pro Arg Ala Phe Asp His Thr Lys Gln
   305 310 315 320
   Asn Gln Asp Trp Thr Gln Trp Asn Pro Ser His Ser Asn Pro Glu Val
   325 330 335
   Leu Leu Gln Thr Thr Thr Arg Met Thr Asp Val Asp Arg Asn Gly Thr
   340 345 350
   Thr Ala Tyr Glu Gly Asn Trp Asn Pro Glu Ala His Pro Pro Leu Ile
   355 360 365
   His His Glu His His Glu Glu Glu Glu Thr Pro His Ser Thr Ser Thr
   370 375 380
   Ile Gln Ala Thr Pro Ser Ser Thr Thr Glu Glu Thr Ala Thr Gln Lys
   385 390 395 400
   Glu Gln Trp Phe Gly Asn Arg Trp His Glu Gly Tyr Arg Gln Thr Pro
   405 410 415
   Lys Glu Asp Ser His Ser Thr Thr Gly Thr Ala Ala Ala Ser Ala His
   420 425 430
   Thr Ser His Pro Met Gln Gly Arg Thr Thr Pro Ser Pro Glu Asp Ser
   435 440 445
   Ser Trp Thr Asp Phe Phe Asn Pro Ile Ser His Pro Met Gly Arg Gly
       450                 455                 460
   His Gln Ala Gly Arg Arg Met Asp Met Asp Ser Ser His Ser Ile Thr
   465                 470                 475                 480
   Leu Gln Pro Thr Ala Asn Pro Asn Thr Gly Leu Val Glu Asp Leu Asp
                   485                 490                 495
   Arg Thr Gly Pro Leu Ser Met Thr Thr Gln Gln Ser Asn Ser Gln Ser
               500                 505                 510
   Phe Ser Thr Ser His Glu Gly Leu Glu Glu Asp Lys Asp His Pro Thr
           515                 520                 525
   Thr Ser Thr Leu Thr Ser Ser Asn Arg Asn Asp Val Thr Gly Gly Arg
       530                 535                 540
   Arg Asp Pro Asn His Ser Glu Gly Ser Thr Thr Leu Leu Glu Gly Tyr
   545                 550                 555                 560
   Thr Ser His Tyr Pro His Thr Lys Glu Ser Arg Thr Phe Ile Pro Val
                   565                 570                 575
   Thr Ser Ala Lys Thr Gly Ser Phe Gly Val Thr Ala Val Thr Val Gly
               580                 585                 590
   Asp Ser Asn Ser Asn Val Asn Arg Ser Leu Ser Gly Asp Gln Asp Thr
           595                 600                 605
   Phe His Pro Ser Gly Gly Ser His Thr Thr His Gly Ser Glu Ser Asp
       610                 615                 620
   Gly His Ser His Gly Ser Gln Glu Gly Gly Ala Asn Thr Thr Ser Gly
   625                 630                 635                 640
   Pro Ile Arg Thr Pro Gln Ile Pro Glu Trp Leu Ile Ile Leu Ala Ser
                   645                 650                 655
   Leu Leu Ala Leu Ala Leu Ile Leu Ala Val Cys Ile Ala Val Asn Ser
               660                 665                 670
   Arg Arg Arg Cys Gly Gln Lys Lys Lys Leu Val Ile Asn Ser Gly Asn
           675                 680                 685
   Gly Ala Val Glu Asp Arg Lys Pro Ser Gly Leu Asn Gly Glu Ala Ser
       690                 695                 700
   Lys Ser Gln Glu Met Val His Leu Val Asn Lys Glu Ser Ser Glu Thr
   705 710 715 720
   Pro Asp Gln Phe Met Thr Ala Asp Glu Thr Arg Asn Leu Gln Asn Val
   725 730 735
   Asp Met Lys Ile Gly Val
   740
<210> 54
   <211> 7
   <212> PRT
   <213> artificial sequence
<220>
   <223> HIV TAT protein PTD derived cell penetrating peptide
<400> 54
   Tyr Gly Arg Lys Lys Arg Arg
   1 5
<210> 55
   <211> 11
   <212> PRT
   <213> artificial sequence
<220>
   <223> HIV TAT protein PTD derived cell penetrating peptide
<400> 55
   Tyr Gly Arg Lys Lys Arg Arg Gln Arg Arg Arg
   1 5 10
<210> 56
   <211> 5
   <212> PRT
   <213> artificial sequence
<220>
   <223> HIV TAT protein PTD derived cell penetrating peptide
<400> 56
   Arg Arg Gln Arg Arg
   1 5